# EUROPEAN PATENT SPECIFICATION

(11) **EP 1 827 678 B1**
(45) Date of publication and mention of the grant of the patent: **18.07.2012**
(21) Application number: 05778890.3
(22) Date of filing: 31.08.2005
(51) Int. Cl.: B01J 19/12, B01J 19/00, B01J 23/44, B01J 37/025, B01J 31/02, B01J 31/04, B01J 31/22, C07C 41/16, C07C 41/30, C07C 43/23, C07C 45/68, C07C 43/205, C07C 43/215, C07C 45/65, C07C 49/794, C07C 49/86, C07C 67/343, C07C 201/12, C07C 205/06, C07C 205/56, C07C 209/02, C07C 213/08, C07C 253/30, C07C 1/32, C07C 51/353

(54) **METHOD AND APPARATUS FOR PERFORMING MICRO-SCALE CHEMICAL REACTIONS**
VERFAHREN UND VORRICHTUNG ZUR DURCHFÜHRUNG VON CHEMISCHEN REAKTIONEN IM MIKROMASSSTAB
PROCEDE ET APPAREIL POUR REALISER DES REACTIONS CHIMIQUES A MICROECHELLE

(30) Priority: 31.08.2004 US 605505 P
(43) Date of publication of application: 05.09.2007
(73) Proprietor: Total Synthesis Ltd., Burlington ON L7T 1A6 (CA)
(72) Inventor: ORGAN, Michael, Burlington, Ontario L7T 1A6 (CA); COMER, Eamon, Malden, MA 02148 (US)
(74) Representative: Harrison Goddard Foote
(86) International application number: PCT/CA2005/001333
(87) International publication number: WO 2006/024167

(56) References cited:
- WO-A1-2004/009231
- WO-A1-2004/009231
- WO-A2-00/07695
- CA-A1- 2 328 944
- DE-A1- 19 834 980
- DE-A1- 19 834 980
- GB-A- 2 325 464
- US-A- 4 204 423
- US-A- 4 204 423
- US-A- 5 415 841

## Description

### FIELD OF THE INVENTION

This invention relates to micro reactor technology (MRT), and to a method and apparatus for performing chemical reactions.

### BACKGROUND OF THE INVENTION

The number of publications in microwave assisted organic synthesis has increased dramatically in recent years. This growth in popularity can be attributed to reduction in reaction times compared to conventional heating methods. Microwave heating has also been reported to increase yields and produce cleaner reactions than traditional heating. A method of performing organic reactions in a continuous flow manner with microwave heating has been developed recently for relatively large (gram scale) organic synthesis

The performance of organic reactions on very small scale in microchannels has many advantages such as the ability to perform high throughput synthesis with a minimum amount of starting materials. Minimizing the required amount of starting materials can be advantageous since the materials can be valuable or, in some cases, hazardous. Microreactor technology can provide benefits for drug discovery by allowing for high throughput screening of a large number of compounds that are quickly available using this method. Additionally, higher yields are reported for some reactions in microreactors compared to larger batch scale synthesis. Traditionally, reactions on microscale quantities have been performed at room temperature in microreactors, however, it would be of considerable advantage if these reactions could be carried out at higher temperatures using microwave heating.

Microwave heating has recently been disclosed in association with microreactors using etched micro-chips. This method generally includes that a metal strip be attached on the outside of the chip to absorb microwave energy, which in turn can transfer the energy (generally as heat) to the reaction. This method can be very costly, since etching of micro-channels in a microchip, as well as attaching the metal strip, can be time-consuming and the metal itself (generally of gold) can be expensive.
It is known from US 4,204,423 to provide a chromatographic column for gas and liquid chromatography in which the column is heated by microwave energy:
It is known from DE 198 34 980 to provide a reactor including capillary tubes in which a reactant is directly heated by microwave energy.

### SUMMARY OF THE INVENTION

The present invention provides a method and apparatus for performing reactions at micro-scale levels that can advantageously use minimal starting components and generate minimal waste. According to some embodiments, the present invention provides reaction capillaries in which a reactant can undergo a reaction to provide a desired product. In accordance with the present invention; reactions performed in capillaries with microwave irradiation can provide a dramatic rate enhancement, illustrating that these small-volume reaction vessels in capillary form are quite able to pick up the 'microwave effect', and can yet avoid some of the drawbacks associated with known microreactors and methods of their use.

The apparatus of the present invention can advantageously use readily available inexpensive/disposable capillary tubes that require no special fabrication. The capillary tubes can be of various sizes with different diameters, which can be selected for respective desired effects on microwave absorption and on factors such as laminar flow. The tubes can be generally straight to reduce or eliminate the risk of blockage. The capillary tubes have an inner film lining to allow for a more efficient heating of a reactant in contact with the film lining. The capillary tubes can include a treatment media supported in the lumens for contacting the reactant and/or product passing through the capillaries. The treatment can be in the form of polymeric balls or granules, coated or infused with one or more treatment compounds. The treatment compounds can include secondary reagents, catalysts, and/or scavengers.

The method of the present invention can facilitate the production of libraries of compounds in a continuous flow manner, i.e. allows for the high throughput continuous production of libraries of compounds. The method can also facilitate the formation of relatively large quantities of products that can be isolated for analysis using standard analytical procedures. Increased quantity of a desired product can be produced by operating the apparatus of the present invention for a longer period of time (i.e. running continuous flow for longer); and keeping the volume of the components as they interact in the reaction generally constant.

The present invention can be particularly well suited for green chemistry in which water is present and which absorbs microwave radiation readily.

In accordance with a first aspect, the present invention provides a reactor apparatus as defined in claim 1 of the appended claims.

The reactor apparatus has an inner surface that is provided with a lining adapted to facilitate the reaction of the reactant. The lining is of a microwave-absorbing material; and can be of a material that provides a chemical catalyst for the reaction. The lining can be of palladium, and can have a thickness of about 6 microns.

The reactor apparatus can include a reactant supply in fluid communication with the lumen of the capillary, and can include a manifold coupled downstream of the reactant supply and upstream of the capillary. The manifold can have at least one outlet port and a plurality of inlet ports in fluid communication with the at least one outlet port. The reactant supply can include a plurality of reagent reservoirs in fluid communication with respective ones of the plurality of inlet ports of the manifold. The reactor apparatus can include a flow inducer for urging the reagent from each reservoir to the respective inlet ports.

The reactor apparatus can be provided with a collection vessel at a downstream end of the reaction capillary for receiving product from the capillary. An analyzer can be provided in fluid communication with the downstream end of the capillary for in-process confirmation of satisfactory reaction of the reactant within the capillary. The reaction capillary can have an axial length extending between upstream and downstream ends, and about 1 cm thereof can be exposed directly to the microwaves. The reaction capillary can have an inner diameter that is less than about 1500 microns.

In embodiments of the present invention, a capillary tube device may provide a reaction chamber, the device having a generally cylindrical wall having an inner surface defining a lumen, and a reaction enhancing film lining the inner surface, the film configured to contact a reactant contained in the device.

The film can be of a material consisting of or including metal, and can be of palladium. The film can have a thickness of between about 2 to about 10 microns, and can be about 6 microns. The lumen can have axially opposed upstream and downstream ends for receiving liquid into and dispensing liquid from the device, respectively, the lumen being generally straight between the upstream and downstream ends.

According to another aspect, the present invention provides a method of reacting a reactant as defined in claim 17 of the appended claims.

The capillary includes a reaction-enhancing film on an inner surface thereof for contacting the reactant passing through the capillary. The microwave energy is absorbed by the film and transferred to the reactant as heat. The film can provide a chemical catalyst for the reaction, can be of palladium, and can be about 6 microns in thickness.

A thin film can be formed on the inner surface by way of a method including preparing a carrier solution containing a desired film material in generally dissolved form; filling a tube with the carrier solution; heating the tube and carrier solution contained therein until the dissolved material has deposited on an inner surface of the tube; and evacuating the solution from the tube.

The method can include heating the emptied tube with the deposited film material thereon. The carrier solution can include palladium acetate, and can include an amount of base solution. The base solution can include potassium hydroxide. During heating of the filled tube, the tube can be periodically re-oriented to promote uniform deposition of the film material on the inner surface of the tube.

Other advantages and features of the present invention may become more apparent as said invention is described in greater detail in the following descriptions and drawings.

### BRIEF DESCRIPTION OF THE DRAWINGS

For a better understanding of the present invention and to show more clearly how it would be carried into effect, reference will now be made by way of example, to the accompanying drawings that show a preferred embodiment of the present invention, and in which:

Figure 1 is a perspective view of a reactor apparatus in accordance with an embodiment of the present invention;

Figure 2 is a schematic view of the apparatus of Figure 1;

Figure 3 is an enlarged view in cross-section of a capillary element of the apparatus of Figure 1;

Figure 4 is a perspective view of a reactor apparatus in accordance with another embodiment of the present invention;

Figure 5 is a schematic view of the reactor apparatus of Figure 4;

Figure 6 is a modified manifold element of the apparatus of Figure 4;

Figures 7a is a photograph of a lining element in accordance with the present invention, taken at 50X magnification;

Figure 7b is a photograph of an edge portion of the lining of Figure 7a, taken at 5000X magnification;

Figures 7c to 7e are photographs of a front surface portion of the lining of Figure 7a, taken at 1500X, 30000X, and 100000X magnification, respectively; and

Figure 8 is a schematic view of another alternate embodiment of a reactor apparatus in accordance with the present invention.

### DETAILED DESCRIPTION OF THE INVENTION

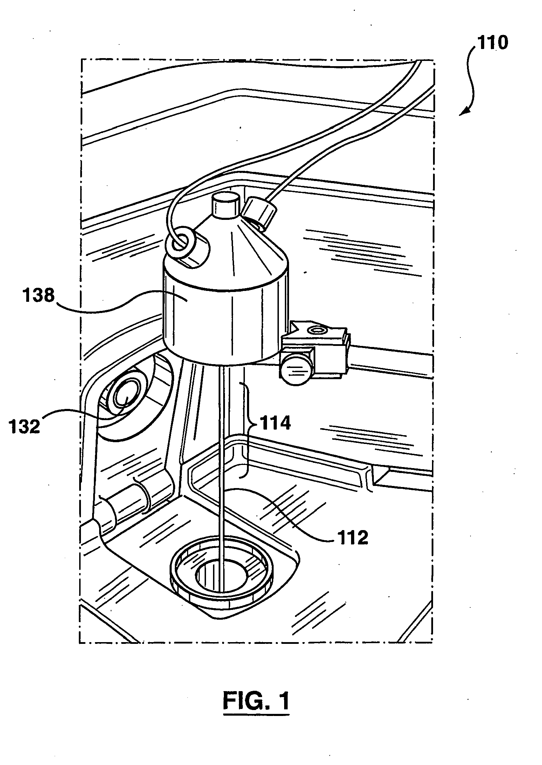

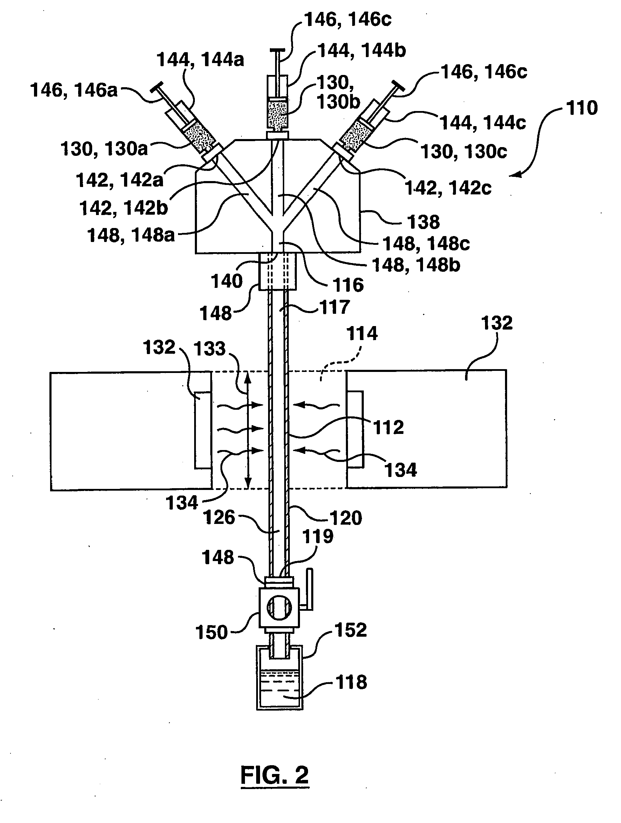

A reactor apparatus 110 in accordance with one embodiment of the present invention is generally shown in Figures 1 and 2. The reactor apparatus 110 includes at least one reaction capillary 112 and a treatment chamber 114 through which at least a portion of the capillary 112 extends.

The capillary 112 can be generally characterized as a fine diameter tube configured to receive a reactant 116. The reactant 116 is generally defined by a selected substance or mixture that is desired to undergo a chemical reaction to produce a product 118. The capillary 112 has an upstream or inlet end 117 for receiving the reactant 116, and a downstream or outlet end 119 for discharging the product 118.

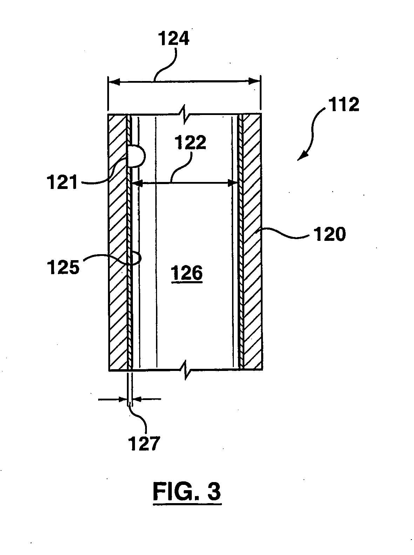

With reference also to Figure 3, in the embodiment illustrated, the capillary 112 has a generally cylindrical wall 120 having an inner surface 121, an inner diameter 122 and an outer diameter 124. The wall 120 is, in the embodiment illustrated, of a glass (or boron silicate) material, although other materials can also be used. The wall 120 is provided with a thin film 125 (also referred to herein as a lining) on its inner surface that can facilitate the reaction in which the reactant 116 produces the product 118. Further details of the film 125 are provided subsequently herein.

The capillary 112 has a generally hollow interior defining a lumen 126 through which the reactant 116 and product 118 can flow. The inner diameter 122 of the capillary 112 is generally small in relation to its length. For example, the inner diameter 122 can be generally less than about 1.5 mm or less than about 2.0 mm. In particular embodiments, capillaries 112 having inner diameters 122 of about 200 microns, of about 380 microns, and of about 1200 microns have been found to perform satisfactorily.

The reactant 116 can include one or more starting materials or input reagents 130. In the embodiment illustrated, the reactant 116 includes a mixture of three input reagents 130 identified as 130a, 130b, and 130c. One or more of the reagents 130 can include a solvent or catalyst. The product 118 can similarly include one or more output components, and can include an amount of unreacted reactant 116.

The treatment chamber 114 is generally adapted to facilitate the initiation and/or progress of the reaction by which the product 118 is produced from the reactant 116. The treatment chamber 114 can be adapted to impart energy to the reactant 116 in the reaction capillary 112 to facilitate the reaction. In the embodiment illustrated, the apparatus 110 includes a magnetron 132 configured to direct microwave energy (identified at arrows 134 in Figure 1) towards the capillary 112 in the treatment chamber 114. The magnetron 132 extends, in the illustrated embodiment, along about 1 cm of the axial length of the capillary 112, defining a treatment chamber length 133. The volume of the lumen 126 of the capillary 112 that is generally located in the treatment chamber 114 defines a reaction chamber. The reaction chamber generally contains a mixture of reactant 116 and product 118, while upstream and downstream of the reaction chamber, mostly only reactant 116 and product 118, respectively, will exist.

To facilitate introduction of the reactant 116 into the inlet end 117 of the capillary 112, the apparatus 110 can be provided with a manifold 138. The manifold 138 has an outlet port 140 that provides a supply of the reactant 116 for the capillary 112. The manifold 138 can have a plurality of inlet ports 142. In the embodiment illustrated, the manifold 138 has three inlet ports 142, identified as 142a, 142b, and 142c for receiving a separate supply of the reagents 130a, 130b, and 130c, respectively. The reagents 130a, 130b, and 130c can be delivered in respective vials 144a, 144b, and 144c that can be coupled to the respective inlet ports 142a, 142b, 142c.

The vials 144 can be in the form of, for example, but not limited to, syringes or commercially pre-filled containers or flasks. The vials 144 can each be coupled to respective flow inducers 146 for urging a respective reagent 130 from the vial 144 to the respective inlet port 142. The flow inducers 146 can be in the form of, for example, but not limited to, peristaltic pumps or syringe pumps (shown schematically in Figure 1).

Each of the inlet ports 142 of the manifold 138 is in fluid communication with the outlet port 140 via respective feed channels 148 (i.e. channels 148a, 148b, and 148c, respectively) extending through the body of the manifold. The manifold 138 can be constructed of a non-reactive material with respect to the reagents 130 and/or reactant 116, and in the embodiment illustrated is of stainless steel construction.

To use the apparatus 110, the reaction components or reagents 130 necessary to perform the desired chemical transformation can be selected and loaded separately into the vials 144. This may require separating the reagents 130 from each other, and can include separation of any components and/or catalysts necessary to make the transformation from reactant 116 to product 118. The particular selection of the various reagents 130 can generally be determined by the nature of the reaction components themselves, the reaction being performed, and/or the application for the reaction. The separate reagents 130 can include a homogeneous or heterogeneous solution; both are generally suitable for use with the apparatus 110.

The vials 144 can then be coupled to the respective inlet ports 142 of the manifold 138, and the flow inducers 146 (e.g. syringe pumps) can be adjusted to provide a desired supply/flowrate.

In some embodiments, the vials 144 can be coupled to the inlet ports 130 using a snap-fit coupler. Alternatively, the vials 144 can be coupled to the inlet ports 130 by other means, such as, for example, tubing. In some cases the manifold 138 may have more inlet ports 130 than the number of vials 144 being used for a reaction, in which case the unused inlet ports 130 can be capped off.

If the capillary 112 is not already in place, the inlet end 117 thereof can be coupled to the outlet port 140 of the manifold 138. A suitable connector for coupling the capillary to the manifold can be, for example, a Microtight^{™} connector, shown generally at 148.

The outlet end 119 of the capillary 112 can be coupled to a collection and/or analysis device as desired. A switching valve 150 can be placed in the effluent stream leaving the capillary to toggle the effluent between, for example, a collection device, an analytical device, and waste. Alternatively, the effluent stream can be split between analysis and collection with an additional setting to direct it to waste. In the embodiment illustrated, the outlet end 119 of the capillary 112 is coupled to a collection vessel 152 that can be in the form of, for example, but not limited to, a test tube, flask, or fraction collector.

If not already so, the capillary 112 can be positioned in the treatment chamber 114. The capillary 112 can be arranged laterally so that the capillary 112 is aligned with a central portion of the magnetron 132.

To initiate the reaction, the syringe pumps 146 can be set into operation at the desired flowrates, and the magnetron 132 can be powered at a desired power setting to deliver the desired amount of energy to the reactant 116 in the capillary 112. As the reactant 116 flows through the capillary 112 in the treatment chamber 114, the reactant 116 is irradiated by the microwaves 134. The flow rates of the pumps 146 and the power settings for the magnetron 132 can be adjusted to heat the reactant 116 an amount that provides optimum yield of the product 118 from the reactant 116.

Once the reaction has started, the first (transient condition) amount of product 118 can be directed to waste via valve 150. Once all non-irradiated material (or otherwise corrupt, pre-steady state material) has exited the capillary 112, the product 118 can be collected in a collection vessel and/or analysed.

The apparatus 110 can thus facilitate production of the product 118 from the reactant 116 in a controlled manner and with high yield. The apparatus 110 can also provide a reaction channel (i.e. the lumen 126) that is generally straight (non-undulating) between the inlet end 117 and outlet end 119 of the capillary 112, so that the risk of blockage of the lumen 126 due to, for example, solidification of the reactant 116 and/or product 118 passing through the lumen 126 is greatly reduced. In the embodiment illustrated, the capillary 112 is oriented generally vertically, with the inlet end 117 positioned vertically above the outlet end 119. The apparatus 110 can be used to provide increased quantities of a desired product 118 by flowing more reactant 116 through the capillary 112, and keeping the size of the reaction chamber constant. Effects of "scaling up" the volumes of the reagents in contact with each other during the reaction are thus avoided.

Variations to the apparatus 110 and its method of use as described above can be made within the scope of the present invention. For example, the reactant 116 can be prepared by mixing reagents 130 in a beaker, for example, withdrawing a desired amount of the reactant 116 in a syringe or vial, and coupling the vial to the inlet end 117 of the capillary 112, so that the manifold 138 is not required. Such pre-mixed reactant 116 can be of heterogeneous or homogenous composition. As another variation, the capillary 112 can be configured in a U-shape, rather than a straight vertical configuration. A U-shaped configuration can increase the exposure of the reactant 116 to the microwaves 134 without increasing the size of the magnetron 132. In another variation, the outlet end 119 of the capillary can be coupled to the inlet of a second apparatus 110 positioned downstream of the first apparatus 110. The second apparatus 110 can use as a reagent 130 the product 118 of the first apparatus. The present invention includes aspects of these or any other variations of embodiments described herein combined separately or in combination with aspects of one or more other embodiments described herein.

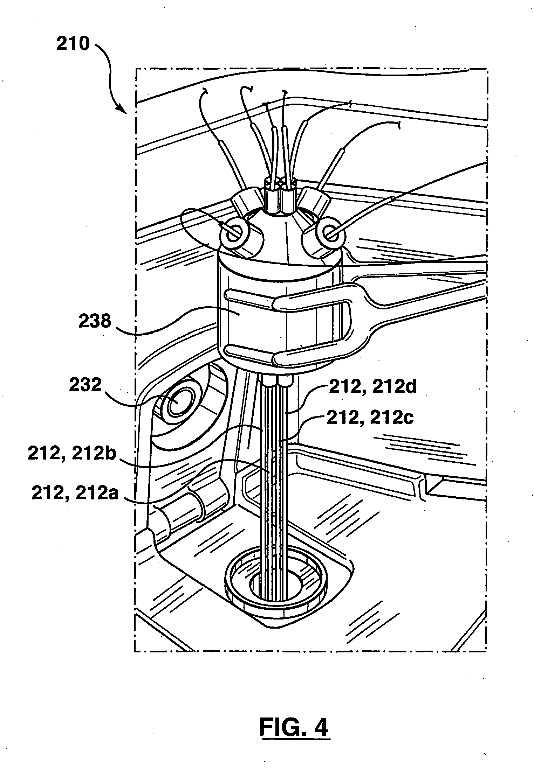

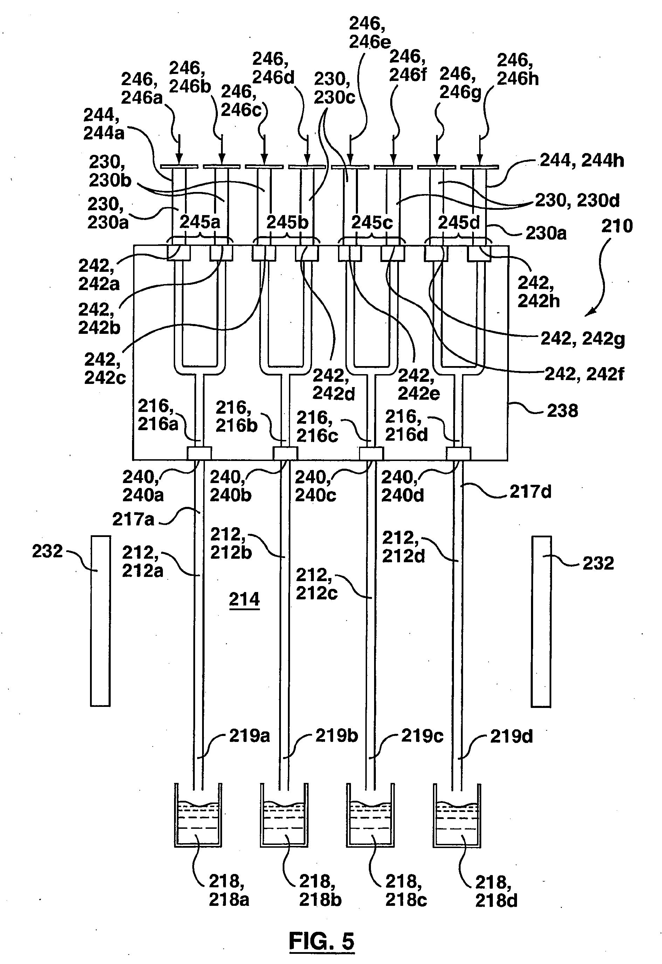

Another embodiment of a reactor apparatus 210 in accordance with the present invention is shown in Figures 4 and 5. The reactor 210 has many similarities to the reactor 110, and like features are identified by like reference characters, incremented by 100.

The reactor 210 has a plurality of parallel reaction capillaries 212 extending through a treatment chamber 214. Each of the plurality of capillaries 212 can receive distinct reactants 216, respectively, so that the reactor 210 can facilitate preparing libraries of distinct products 218 simultaneously by parallel capillary microwave irradiation.

The reactor 210 can include a manifold 238 having a plurality of outlet ports 240, each one of which can be coupled to a respective one of the plurality of reaction capillaries 212. The manifold 238 can have a plurality of inlet ports 242.

In the embodiment illustrated, the manifold 238 has eight inlet ports 242, identified as inlet ports 242a-242h. The manifold 238 has four outlet ports 240, identified as outlet ports 240a, 240b, 240c, and 240d. The apparatus 210 has four parallel reaction capillaries 212, identified as 212a, 212b, 212c, and 212d. Each capillary 212 has a respective inlet end 217 coupled to a respective one of the outlet ports 240.

As best seen in Figure 5, in the embodiment illustrated, the inlet ports 244 are arranged in four inlet port pairs 245a, 245b, 245c, and 245d. Each inlet port 244 in one pair 245 is in fluid communication with a common one of the four outlet ports 240. For example, the inlet port pair 245a include inlet ports 242a and 242b, each of which are in fluid communication with the outlet port 240a. The inlet port pair 245d include inlet ports 242g and 242h, each of which are in fluid communication with the outlet port 240d. In this way, two distinct reagents 130 can be combined to form a respective one of the reactants 116 being supplied to a respective reaction capillary 212.

Eight distinct reagents 130 can be coupled to respective ones of the inlet ports 242a-242h. Alternatively, one or more inlet ports 242 in different pairs of ports can share a common reagent 230. In the embodiment illustrated, four reagents 230a, 230b, 230c, and 230d are provided. Each pair 245 of inlet ports 242 is supplied with a distinct combination of two of the four reagents 230a, 230b, 230c, and 230d. In particular, for the illustrated embodiment, inlet ports 242a and 242b are supplied with reagents 230a and 230b, respectively, which combine to form reactant 216a at outlet port 240a. Inlet ports 242c and 242d are supplied with reagents 230b and 230c, respectively, which combine to form reactant 216b at outlet port 240b. Inlet ports 242e and 242f are supplied with reagents 230c and 230d, respectively, which combine to form reactant 216c at outlet port 240c. Inlet ports 242g and 242h are supplied with reagents 230d and 230a, respectively, which combine to form reactant 216b at outlet port 240b.

This method can be used to produce compounds (products 218) in successive multiples of four. This can facilitate the simultaneous generation of libraries of distinct products 218 that have some reagents in common. Simultaneous generation of the products 218 can ensure that each distinct product 218 has been produced under similar operating conditions, which can facilitate subsequent comparative use of the products 218. Preparing multiple products (four in the embodiment illustrated, but many more capillaries could also be provided) can also greatly reduce the amount of time required to prepare a desired collection of products.

In use of the reactor apparatus 210, separated reagents 230 are supplied to the inlet ports 242 of the manifold 238. Each reagent 230 is combined with one or more other reagents 230 to provide distinct reactants 216. The reactants 216 are delivered to the parallel reaction capillaries 212 where they react while being irradiated.

The method of using the reactor apparatus 210 is similar to the method of using the apparatus 110. The method includes selecting the reaction components (i.e. reagents 230) necessary to provide the four products 218a-218d, including the appropriate solvent, reactants, catalysts, etc., and loading them separately, as necessary, into separate vials 244. In the embodiment illustrated, the two reagents 230 that react to form the desired product 218 for each reaction capillary 212 will be in separate, but paired vials 244.

The flow inducers 246 can be adjusted to provide the desired supply/flowrate of the reagents 230. The reaction capillaries 212 can be connected to the outlet ports 240 of the manifold 238 using the appropriate connectors.

The outlet end 219 of the reaction capillaries 212 can be connected to a collection or analysis device as required. A switching valve can be placed in the effluent streams from each capillary 212 to toggle the effluent between a collection device, an analytical device, and waste. Alternatively, the effluent stream can be split between analysis and collection with an additional setting to direct it to waste.

The flow inducers 246 can be activated and the magnetron 232 can be energized at the desired power settings to deliver the desired/optimized microwave energy to the reactants 216 in the reaction capillaries 212.

Based on the volume of the capillaries and flowrate, collection and/or analysis of the products (effluent) from the bottom of the capillary 212 can begin, once all non-irradiated material is known to have cleared the capillary entirely.

Once a sufficient quantity of the desired products 218 has been collected, the vials 244 can be replaced with by a second group of vials containing the reagents for making a second batch of four products.

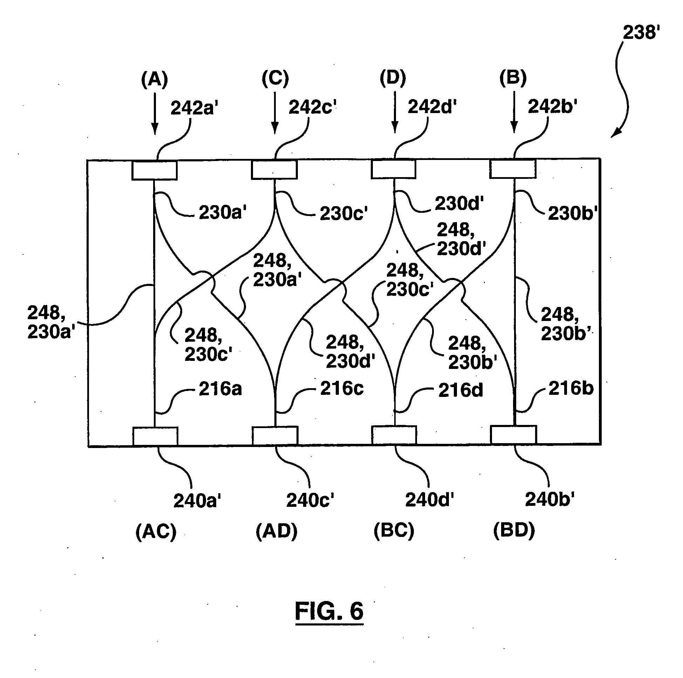

The apparatus 210 and method of its use can be varied within the scope of the present invention. For example, as seen in Figure 6, a modified manifold 238' can be used in place of the manifold 238. In the modified manifold 238', each inlet port 242' can be in fluid communication with more than one outlet port 240', and each outlet port 240' can be in fluid communication with more than one inlet port 242'. In the embodiment illustrated, the modified manifold 238' has four active inlet ports 242' (rather then eight), identified as inlet ports 242a', 242b', 242c', and 242d'. Each of the four inlet ports 242' can be in fluid communication with two of the outlet ports 240'. For example, the inlet port 242a' can be in fluid communication with the outlet ports 240a' and 240c'. The inlet port 242b' can be in fluid communication with outlet ports 240b' and 240d'. The inlet port 242c' can be in fluid communication with outlet ports 240a' and 240d'; and, the inlet port 242d' can be in fluid communication with outlet ports 240b' and 240c'.

In such an alternative embodiment, each of the inlet ports 242a'-242d' is adapted to be coupled to a respective reagent reservoir or vial 244a'-244d', each containing a respective reagent 230a'-230d'. Each capillary 212a-212d thus receives a respective reactant 216a-216d and produces a respective product 218a-218d. For example, the capillary 212a receives reactant 216a (from outlet port 240a') and dispenses product 218a. The reactant 216a includes reagents 230a and 230c. The capillary 212d receives reactant 216d (from outlet port 240d') and dispenses product 218d. The reactant 216d includes reagents 230b and 230c.

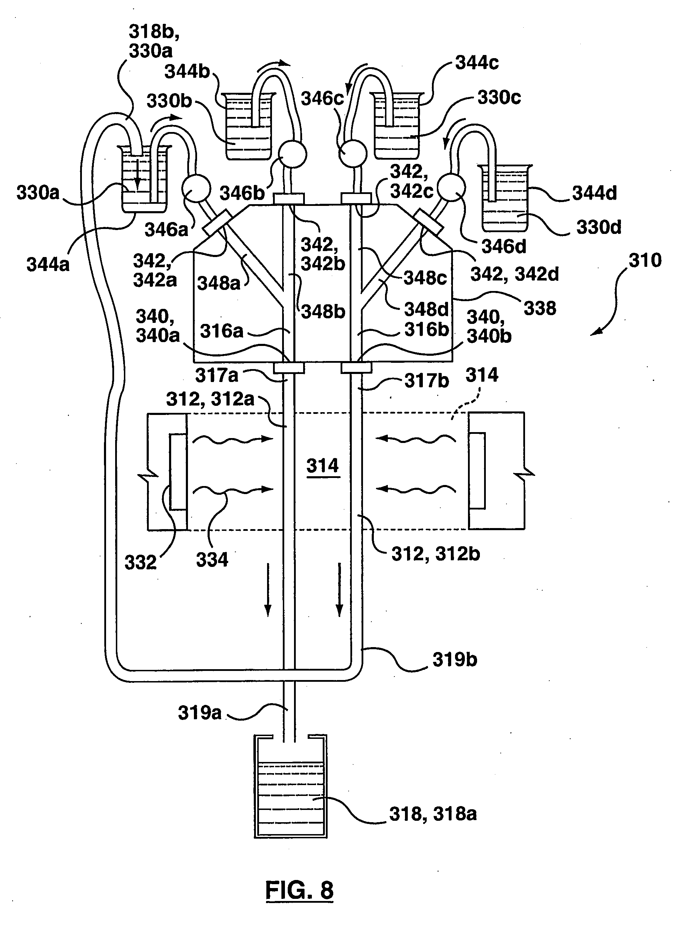

Another embodiment of a reactor apparatus 310 can be seen in Figure 8. The apparatus 310 is similar to apparatus 210, and like features are identified by like reference characters, incremented by 100. In apparatus 310, the outlet ends 319 of one or some of the reaction capillaries 312 can be coupled to one or some of the inlet ports 342 of the manifold 338. In this way, a product 318 from a capillary 312 can serve as a reaction intermediate that can be fed back into the manifold 338 as a reagent 330. Such a configuration can provide automated multi-step microwave-assisted synthesis functionality.

Further details of the film lining 125 will now be described. The lining 125 can be provided on any one or more of the capillaries 112, 212, described above. As well, although described herein in relation to the capillaries 112, 212, the present invention comprehends that the lining 125 and methods of making such a lining 125 can be used in applications other than for capillaries 112, 212.

The lining 125 is generally in the form of thin layer or coating of material provided on the inner surface of the capillaries. The lining 125 is of a metal or metal-containing material that readily absorbs energy from the microwaves 134, and can store and transfer this energy (generally in the form of heat) to the reactant 116 in contact with the lining 125. The lining 125 can also be of a material that serves as a chemical catalyst for the reaction taking place within the reaction capillary 112, 212. Suitable materials for the lining 125 can include, but not limited to, palladium, silver, copper, nickel, gold, rhodium, and/or platinum.

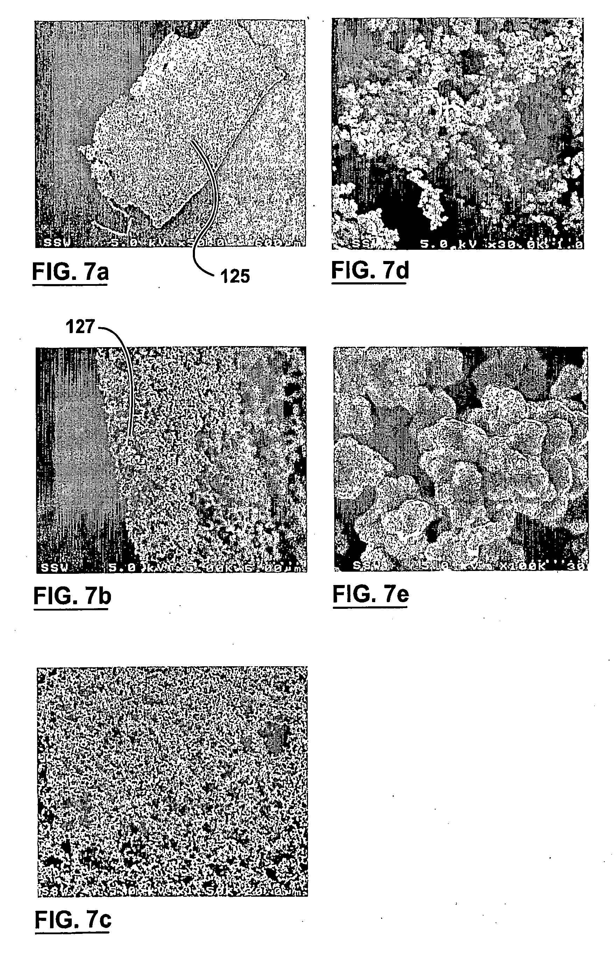

Referring now also to Figures 7a-7e, in one embodiment, the lining 125 is of elemental palladium. The lining 125 can have a thickness 127 of about 2 microns to about 10 microns, or generally less than about 15 microns. Such a lining 125 has been found to satisfactorily act as a catalyst in many reactions, and to absorb energy from the microwaves 134 and transfer this as heat to the reactant 116 in the capillary 112. The lining thickness 127 should be kept sufficiently thin to prevent arcing of the microwaves 134, and to prevent melting of the lining 125.

The lining 125 can have a relatively high porosity (about 75%), and the porosity can be generally uniform (Fig. 7c). The film lining 125 can include small grains that are of a size of about 40 to 60 mm in diameter (Fig. 7d and 7e). For the sample shown in Figure 7a; the thickness 127 of the film 125 is about 6 microns (edge shown in Fig. 7b).

EDX analysis was performed on the film formed in the capillary as well as on films that were prepared on glass plates. The linings 125, after the solution has been drained, but prior to calcinations, contained an average of 28 wt% of carbon while for the calcinated sample this was lower, having a value of about 15 wt%. For the capillary 112 this amount was lower down to 5.5 wt%. This can be explained by the increase in porosity that allows trapped carbonaceous material to be removed more efficiently. The films prepared in the capillary included a majority of Pd (about 94.0 wt%) and only about 0.3 wt% of oxygen was detected. This can be a result of the presence of a thin oxide film on the Pd. No other elements were detected. The presence of such a small amount of carbon and oxygen indicates that the film is mostly metallic.

Using the capillary weight change before and after the film preparation, film thickness, and the dimension of the capillary, it was possible to evaluate the density of the Pd film to be about 3 mg/cm³. This would correspond to a porosity of about 75%.

In summary, the films 125 prepared according to the method of the present invention (described further hereinafter) can be highly porous and composed of nanometer size grains (94.0 wt% Pd and 5.5 wt% carbon). The film thickness can be about 6 microns and the film porosity can be of the order of 75%.

In accordance with the present invention, the following method is provided for producing the lining 125. A 0.1 mmol/mL stock solution of palladium acetate in DMF or DMA is prepared. An amount of the stock solution can be mixed with a base solution, to provide a carrier solution. For example, 1.0 mL of the stock solution can be mixed with 0.2 mL of a base solution in the form of an aqueous solution of potassium hydroxide (2M) in a vial, forming a carrier solution. The base (potassium hydroxide) is optional and can increase the rate of metal deposition.

The internal surface of the capillary 112 is cleaned using a 10% aqueous solution of hydrofluoric acid. An amount of 1.0 mL of the intermediate solution is taken up in a 1.0 mL syringe. In place of a needle extending from the syringe, a capillary is coupled to the neck of the syringe by, for example, wrapping tape around the end of the capillary to ensure they are coupled together in leak-proof fashion.

The carrier solution is then introduced into the capillary so that the lumen 126 is generally filled along at least a portion of its axial length. The inlet and outlet ends 117 and 119 can be plugged using tape or septa.

The filled capillaries 112 can then be placed on a metallic tray and put inside of a laboratory thermal furnace. In accordance with one embodiment of the present invention, the temperature of the furnace can be raised gradually and then kept constant at about 120 °C to 160 °C for about 30 to 120 minutes.

The palladium, which can begin to release almost immediately from the carrier solution, starts to deposit gradually on the inner surface 121 of the capillary 112. The capillaries 112 can be rolled several times to facilitate uniform coating.

After the coated capillaries are removed from the furnace, the residual solution inside the capillaries can be evacuated. The temperature of the furnace is then raised to 350°C - 400°C. The capillaries are placed back inside the furnace and calcinated up to 1 minute. This calcination step can be repeated, and in the embodiment illustrated, was repeated twice (total of three calcination treatments). This can help to ensure increased porosity, the removal of residual organic material, and a firmer adhesion of the metal film 125 to the glass surface 121.

The coated capillaries 112 can then be transferred to a clean airtight test tube under argon atmosphere for safe storage until required for use.

Variations of the above method can be made within the scope of the invention. For example, the palladium coating 125 of the capillaries 112 can also be carried out using a carrier solution that has not been mixed with a base solution, or that is generally the same as the stock solution. Without a base, it can take longer for palladium to be released from the carrier solution.

The effect of the "baking" of the film morphology was explored. It is apparent that heating the sample lining 125 to about 350°C to 400°C for about 3 minutes causes the porosity of the film 125 to increase. This seems to indicate that residual carbonaceous material is mostly located on top of the Pd grains ("baking" temperature is well below Pd annealing temperature). We have noted however that the film morphology is more compact when the films are prepared on flat glass plates. This is thought to result from the different film geometrical configurations, namely, that a smaller amount of solution is used in the preparation of the capillary lining 125 (capillary 112 filled with fixed amount of solution) while the plate is immersed in a larger amount of solution.

The film preparation for morphology and composition analysis was identical to the one used in the microwave synthesis. The capillary was cleaved and pieces of the films were fixed on a carbon tape for analysis. Films were also prepared on 1 cm² flat glass substrates bit dipping the glass in the preparation solution. Sample imaging was carried out with an Hitachi S-4500 field emission Scanning Electron Microscopy (SEM) equipped with EDAX Phoenix model energy dispersive x-ray (EDX) analyzer. EDX analysis can detect all elements above atomic number 5 and has a minimum detection limit of 0.5 wt% for most elements. A 5kV electron beam was used to obtain SEM images and EDX spectra. Both the lower and upper SE detectors were used for imaging purposes.

The film morphology was analyzed using Scanning Electron Microscopy (SEM) and Energy Dispersive x-ray (EDX) analyzer. The images presented here were obtained from a piece of the film that was removed from the capillary wall (see Fig. 7a). Figures 7a to 7e show the film morphology for increasing magnification (see figure caption).

The reactor apparatus 110, 210, 310 of the present invention can be applied to the concept of flowing a solution containing starting materials through a vessel that is loaded with secondary reagents, catalysts, and/or scavengers to facilitate organic synthesis offers. This can have huge potential for industries based on synthetic chemistry. Increasingly, such approaches are starting to show up in the chemical literature, although the flow concept, in one form or another, in larger scale industrial synthesis is not new. In such a scheme, secondary reagents, catalysts and/or scavengers are immobilized in the vessel on either a solid support or on the sides of the vessel itself.

According to the present invention, the loaded vessel can be a tube of some sort, and can be a capillary 112 having a treatment compound supported within the lumen 126, for contacting the reactant 116 and/or product flowing through the lumen 126. Axially spaced-apart ends of the capillary 112 can be fritted such that the treatment is contained within the capillary 112, but solutions containing dissolved starting materials (i.e. components of the reactant 116) can readily flow through, reacting as they do. The movement of the starting material solutions (also referred to herein as primary reagents) through the vessel can either be uninterrupted, continuous flow, or it can be by stop flow. In a continuous flow situation, the reactions involved should be fast enough to complete before the starting materials traverse the axial extent of the capillary. For slower conversion, a plug of starting materials (or primary reagents) can be moved into the vessel or capillary 112, held there for a period of time to allow the reaction to complete and then be discharged from the vessel.

The development of smaller scale, flow-through synthetic systems can have a major impact on areas such as the pharmaceutical and agrochemical industries, where the idea is generally not to produce large quantities of any one compound, but rather to produce larger numbers of single compounds to screen for desirable activity. The advantages of small-scale flow synthesis using immobilized reagents, catalysts, or scavengers in this type of application are many.

For example, any unused secondary reagent, and ideally any reagent byproducts, the catalyst, and/or the scavenger, remain attached to the support after they have reacted and generally do not contaminate the product effluent leading to compounds that are, ideally, pure enough to screen without additional purification. The savings in terms of time, consumables, and waste production from the large-scale purification of hundreds or thousands of compounds can result in huge savings to industry and may reduce any negative impact on the environment from such activities.

Vessels filled with the appropriate supported compounds can be produced on large scale very cheaply and can therefore be viewed as a consumable and disposable commodity to the industrial or academic scientist. There would be enormous savings potential in terms of cost in setting reactions up by chemists who instead can buy the vessel ready to be used.

The potential exists to set up consecutive vessels to facilitate in-line synthesis and purification (where necessary) and to be able to perform reactions in sequence so that several transformations can be conducted in one overall operation. Here the product effluent from one reaction vessel can flow, as necessary, through a purification vessel loaded with scavengers, and then into the next reaction vessel to conduct another chemical transformation. This can be repeated as often as necessary until all transformations have been completed.

### Description of Reagent-filled Capillary Devices Designed Specifically For Applications to Microreactor Microwave Application:

According to the present invention, the capillaries 112 can include one or more treatment compounds retained in the lumen 126. The treatment compounds can be immobilized inside of the capillary, either on a solid support that resides in the lumen of the capillary or on the wall of the capillary itself. The treatment compounds can include one or more of a secondary reagent, a catalyst, or a scavenger. The terms secondary reagent, catalyst, and scavenger are described below.

A secondary reagent is defined as a chemical entity that reacts with a starting reactant (or primary reagent) during a synthetic procedure and is consumed in the process to produce a desired product. Atoms from the secondary reagent may, or may not be incorporated into the product of that reaction, but the secondary reagent is consumed in the procedure. If used in excess relative to the molar quantity of the starting reactant, and it is not otherwise consumed in the transformation, residual reagent will be in the reaction mixture upon completion.

A catalyst is a -chemical entity, which can- be -organic or inorganic in nature, that is helpful and/or necessary to effect a chemical transformation where a starting component, and possibly additional reagents, are converted to a desired product. The catalyst is generally not consumed or destroyed and, although parts of the catalyst may be incorporated in the process, it is returned intact or regenerated after each turnover of starting reactant to product.

A scavenger is a chemical entity that is added to a chemical reaction, typically when the reaction is judged complete, to purify the product from residual reactants and/or reagents, catalysts, or other possible reaction byproducts so that the product is obtained in relatively pure form. The scavenger is typically attached to some sort of a solid medium, or to the wall of the vessel, such that the product can be obtained by simple filtration. In some cases, multiple scavengers are required that can either be added to the crude reaction mixture together, or one after another separated by filtration steps to remove the preceding scavenger and its associated scavenged material from the transformation. The pharmaceutical industry generally considers a product that is greater than 80% pure to be suitable for early stage biological screening. As methods and scavengers improve, this industrial standard will increase and some companies will now only screen material that is greater than 90% pure. Although analytically pure products are always desirable for such screening, it is recognized that the time and waste involved in large-scale chromatographic purification of every single product in a large collection, called a chemical or molecular library, is prohibitive from a cost and environmental point of view.

### Nature of the Immobilization:

The treatment compounds can be immobilized in a number of fashions. Immobilizations to the capillary wall itself can be done by laying down a coating on the glass surface that these chemical entities can bond, adhere or coordinate to, or to bond these chemical entities directly to the glass itself. One such example would be the metal films 125 described previously, where the metal film adheres to the surface of the glass and can serve as a chemical catalyst to convert starting reactants to desired products.

In another embodiment, the treatment compounds can be attached, either by an ionic, coordinate and covalent bond, to a matrix that fills the capillary that is sufficiently porous to allow adequate flow as not to create undesirable back pressure on the system. Here, the treatment compound can be attached to the smaller building blocks that form the matrix, or they can be attached to the matrix after it is formed inside the capillary. The matrix is either held in the capillary by its association with the glass wall of the capillary, or by a frit, or by both. One such example of a matrix would be sol-gel derived porous glass (silica).

In another embodiment, the treatment compound can be attached to a treatment media retained in the lumen of the capillary and of sufficient size that it can be held within the capillary by a frit or by a sufficient narrowing of the end of the capillary. The treatment media can be a solid entity, such as, for example, but not limited to, organic polymeric beads (both swelling and non-swelling), porous and non-porous glass beads, silica gel of any mesh size, inorganic supports such as clays, or organic supports such as graphite. In these cases, the treatment compound(s) can be loaded/bonded onto the solid supported material outside of the capillary and then loaded into it. Alternatively, the treatment media can be first loaded into the capillary, and then a solution containing the treatment media can be flowed into the capillary where they become loaded onto the media.

### Description of a Typical Operation/Setup Using a Filled Capillary:

Capillaries that have been supplied with the appropriate treatment compound can be installed in the apparatus 110, 210, 310. Operation of the device with the filled capillaries generally follows similar protocols as outlined above in the DETAILED DESCRIPTION OF THE INVENTION section in terms of capillary attachment to device 138 and 238, the attachment of reactant (130) vials, and flow of the solution containing the reactant and/or additional reagents, as necessary as determined by the chemistry that needs to be conducted, through the capillary while it is being irradiated with microwave irradiation.

### Reagent Filled Capillaries:

In one scenario, the starting component (or primary reagent) 130 can be loaded into a vial (144) with a suitable solvent and is infused through a capillary loaded with a supported secondary reagent necessary to complete the desired chemical transformation. The supported secondary reagent can contain atoms that become incorporated into the product. In this case, there is generally no need to include additional reagents in the same solution with the starting component 130, or in a separate vial that merges with the starting component 130 when it enters the manifold 138.

There may, or may not be the need to include a catalyst in with the reactant 116 in order to effect its transformation with the supported secondary reagent. If there are additional inlet ports in the manifold that merge with this the reaction flowing through the reagent-filled capillary is irradiated in the microwave chamber (312) and the effluent can be directly collected in a collection device (118). Further, the effluent can be sent to an analytical device to measure conversion to product, or the effluent stream can be split to flow both to a collection device and to an analytical station.

In a second scenario, the reaction is set up as detailed above, but additional reagents or catalysts are necessary to complete the reaction that are best kept separate until they are mixed in the manifold (138), immediately prior to entering the reagent-filled capillary. The flow process is started and the conjoined flows flow through the capillary while being irradiated by microwave irradiation. The product effluent is then processed as detailed above.

### Catalyst Filled Capillaries:

In this case, the starting components 130 that are necessary for the chemical transformation are loaded into one or more vials attached to the manifold (138) and flowed through a solid-supported or capillary wall-supported catalyst. The flow process is started and the conjoined flows flow through the capillary while being irradiated by microwave irradiation. The product effluent is then processed as detailed above.

### Scavenger Filled Capillaries:

Scavenger-filled capillaries are used primarily to purify a product mixture. Such a mixture could be formed by a flow method, or a batch prepared method where the material was produced in a single flask without flow. Microwave heating pushes the scavenging process more quickly to completion and leads to cleaner product mixtures. So, a scavenger-filled capillary can be attached to a manifold, such as 138, or not, and the product mixture is flowed through this capillary while being irradiated with microwave irradiation and the product handled as described above.

### Sequential Filled Capillary Operations:

As mentioned previously in this patent application, operations can be set up in a queued fashion, one after another, to perform multiple-step organic transformations. The same can be applied to the filled capillaries. This can be the case for sequential secondary reagent-filled capillaries, catalyst-filled capillaries, and any combination of the two. Also, in-line chromatography can be carried out as well by linking these reagent or catalyst-filled capillaries to a scavenger-filled capillary, thus completing one or more chemical transformation steps and chromatography.

### Parallel Sequential-Filled Capillary Operations:

All of the above mentioned procedures involving the single reaction capillary reactor system can also be conducted in parallel to produce multiple products simultaneously.

While preferred embodiments of the invention have been described herein in detail, it is to be understood that this description is by way of example only, and is not intended to be limiting. The full scope of the invention is to determine from reference to the appended claims.

### EXAMPLES

Some representative examples of the classes of reactions performed using methods in accordance with the present invention are described below.

The following standard abbreviations are used throughout the Examples:
- DMF: N,N-Dimethylformamide
- DMSO: Dimethylsulfoxide
- THF: Tetrahydrofuran
- RBF: Round Bottom Flask
- RT or rt: Room temperature
- TBAF: Tetrabutylammonium flouride
- Fmoc: 9-fluorenylmethoxycarbonyl
- equiv.: equivalent(s)
- cat.: catalyst
- h: hour(s)

All conversions were determined by ¹H NMR and represent the quantity of product relative to starting material. Thus, in reactions where the conversion is reported as 80% the remainder of the material is starting material. All reactions were preformed in a continuous flow mode. The method of operation involved first priming the system with the solvent of choice, then the reaction mixture was continuously flowed through the system with the aid of a syringe pump while heating at a constant power level with a constant microwave irradiation.

### Example 1: Suzuki Reaction Using One inlet Stream an example that does not specifically embody the claimed invention

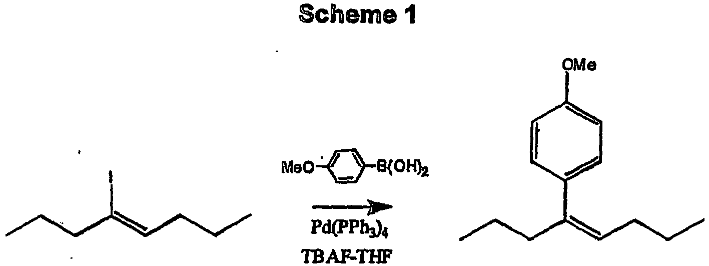

| ***Entry*** | ***Power*** | ***Capillary Diameter*** | ***Flowrate*** | ***Solvent*** | ***Conversion*** |
|---|---|---|---|---|---|
| **1** | 100 W | 200 µm | 2 µl/min. | THF | 100% |
| **2** | 160 W | 200 µm | 5-40 µl/min | THF | 65% |
| **3** | RT | RBF | Batch rxn control | THF | 0% |

| | | | | | |
|---|---|---|---|---|---|
| **Scheme 1 conditions**: 1 equiv. of vinylhalide, 1.1 equiv. boronic acid, 5 equiv. of base, 5 mol% Pd(PPh₃)₄ in THF. Batch rxn control refers to the identical reaction performed under similar conditions (i.e. same concentration) using "standard" chemical techniques (i.e. round bottomed flask with stirrer) at room temperature. | | | | | |

### Example 2 an example that does not specifically embody the claimed invention

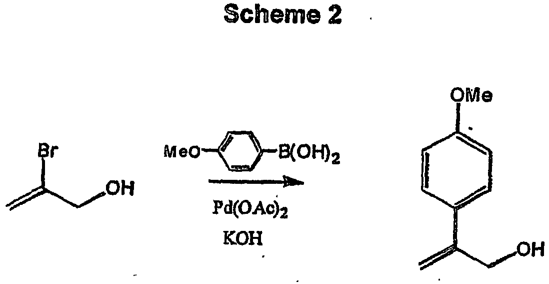

| ***Entry*** | ***Power*** | ***Capillary Diameter*** | ***flowrate*** | ***Solvent*/*base*** | ***Conversion*** |
|---|---|---|---|---|---|
| **1** | 170 W | 380 µm | 40 µl/min | DMF/H₂O | 43% |
| **2** | 150 W | 380 µm | 40 µl/min | DMF/H₂O | 39% |
| **3** | RT | RBF | Batch rxn control. | DMF/H₂O | 0% |

| | | | | | |
|---|---|---|---|---|---|
| **Scheme 2 conditions**: 1 equiv. of vinylhalide, 1.2 equiv. boronic acid, 3 equiv. of base, 5 mol% Pd(OAc)₂ in DMF/H₂O. | | | | | |

### Example 3

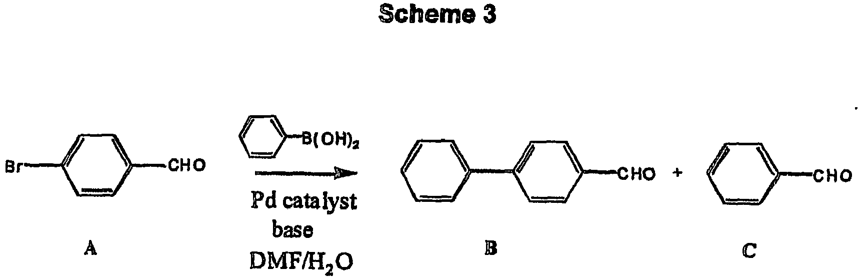

| ***Entry*** | ***Power*** | ***Capillary Diameter*** | ***flowrate*** | ***Base*/*Catalyst*** | ***Ratio A.B.C*** |
|---|---|---|---|---|---|
| **1** | 160 W | 200 µm | 40 µl/min | K₂CO₃ Pd(OAC)₂ | 93(A):7(B) |
| ***2** | 90°C 2h, 60°C 14h. | RBF | Control | K₂CO₃ Pd(OAc)₂ | 100 (B) |
| ****3** | 170 W | 200 µm | 40 µl/min | KOH Pd(OAc)₂ | 37(A):48(B):15(C) |
| ****4** | 170 W | 380 µm | 40 µl/min | KOH Pd(OAc)₂ | 11(A):54(B): 35(C) |
| **5** | 170 W | 380 µm | 40 µl/min | K₂CO₃ Pd(PPh₃)₄ | 26(A):74(B) |

| | | | | | |
|---|---|---|---|---|---|
| **Scheme 3 conditions**: 1 equiv. of arylhalide, 1.2 equiv. boronic acid, 3 equiv. of base, 5 mol% Pd catalyst in DMF/H₂O. Ratio A:B:C represents the ratio of the 2 products B and C relative to starting material A as determined by ¹H NMR. In entries were A and C are not specified, they were not observed. * Entry 2 refers to the identical reaction performed under similar conditions (i.e. same concentration) as in entry 1, using "standard" chemical techniques (i.e. round bottomed flask with stirrer) at 90°C for 2h then at 60°C for 14h with the aid of an oil bath. ** These reactions were performed with a capillary tube which was coated internally with palladium (i.e. capillary 112 with lining 125). | | | | | |

### Example 4 an example that does not specifically embody the claimed invention

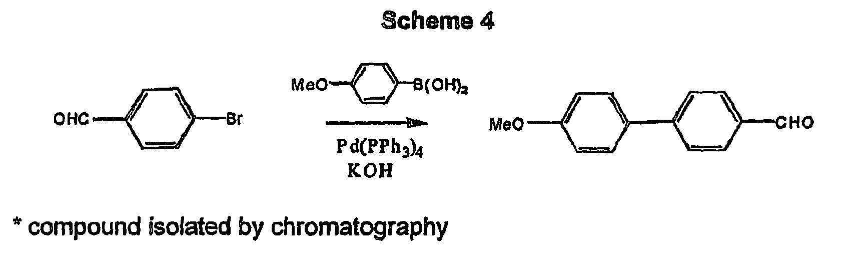

| ***Entry*** | ***Power*** | ***Capillary Diameter*** | ***Flowrate*** | ***Solvent*** | ***Conversion*** |
|---|---|---|---|---|---|
| **1** | 170 W | 380 µm | 40 µl/min | DMF/H₂O | *91% |
| **2** | RT | RBF control | Batch rxn | DMF/H₂O | 32% |

| | | | | | |
|---|---|---|---|---|---|
| **Scheme 4 conditions**: 1 equiv. of arylhalide, 1.2 equiv. boronic acid, 3 equiv. of base, 5 mol% Pd(PPh₃)₄ in DMF/H₂O. | | | | | |

### Example 5 an example that does not specifically embody the claimed invention

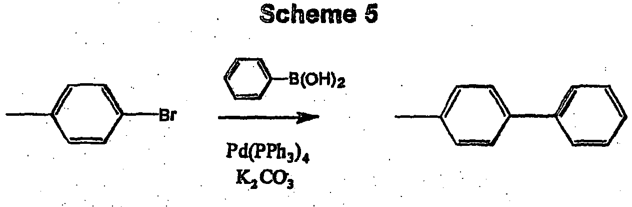

| ***Entry*** | ***Power*** | ***Capillary Diameter*** | ***Flowrate*** | ***Solvent*** | ***Conversion*** |
|---|---|---|---|---|---|
| **1** | 170 W | 380 µm | 40 µl/min | DMF/H₂O | *55% |
| **2** | RT | RBF | Batch rxn control | DMF/H₂O | 34% |

| | | | | | |
|---|---|---|---|---|---|
| * compound isolated by chromatography **Scheme 5 conditions**: 1 equiv. of arylhalide, 1.2 equiv. boronic acid, 3 equiv. of base, 5 mol% Pd(PPh₃)₄ catalyst in DMF/H₂O. | | | | | |

### Example 6

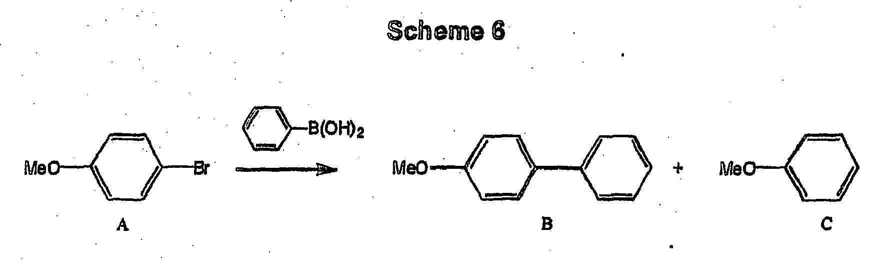

| ***Entry*** | ***Power*** | ***Capillary Diameter*** | ***Flowrate*** | ***Solvent*/ *Catalyst*/ *Base*** | ***Ratio A:B:C*** |
|---|---|---|---|---|---|
| ***1** | 170 W | 380 µm | 25 µl/min | DMF/H₂O, Pd(OAc)₂, KOH | 30(**A**):17(**B**): 53(**C**) |
| ****2** | RT | RBF | Control | DMF/H₂O Pd(OAc)₂, KOH | 0% |
| **3** | 170 W | 380 µm | 25 µ/min | THF, Pd(PPh₃)₄, TBAF | 28(**A**):17(**B**) 55(**C**) |
| *****4** | RT | RBF | Control | THF, Pd(PPh₃)₄, TBAF | 0% |
| **5** | 80°C | RBF | Control | THF, Pd(PPh₃)₄, TBAF | 12 min, 100(**A**):0(**B**) |
| | | | | | 22 min, 100(**A**):0(**B**) |
| | | | | | 1.5 h, 100(**A**):0(**B**) |
| | | | | | 18 h, 0(**A**):100(**B**) |

| | | | | | |
|---|---|---|---|---|---|
| **Scheme 6 conditions:** 1 equiv. of arylhalide, 1.2 equiv. boronic acid, 3 equiv. of base, 5 mol% Pd catalyst. Ratio A:B:C represents the ratio of the 2 products B and C relative to starting material A as determined by ¹H NMR. In entries were C is not specified, it was not observed. * This reaction was performed with a capillary tube which was coated internally with palladium. ** Entry 2 refers to the identical reaction as entry 1, performed under similar conditions (i.e. same concentration) using "standard" chemical techniques (i.e. round bottomed flask with stirrer) at room temperature. *** Entry 4 refers to the identical reaction as entry 3, performed under similar conditions (i.e. same concentration) using "standard" chemical techniques (i.e. round bottomed flask with stirrer) at room temperature. | | | | | |

### Example 7

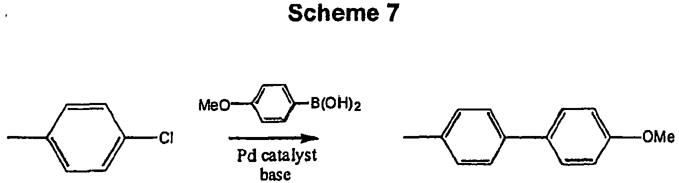

| ***Entr y*** | ***Power*** | ***Capillary Diameter*** | ***Flowrate*** | ***Solvent*/ *catalyst*** | ***Conversion*** |
|---|---|---|---|---|---|
| **1** | 170 W | 380 µm | 25 µl/min | DMF/H₂O Pd(PPh₃)₄ | 0% |
| **2** | 170 W | 380 µm | 25 µl/min | DMF/H₂O Pd(PPh₃)₄ | 0% |
| ****3** | 170 W | 380 µm | 25 µl/min | DMF/H₂O Pd(OAC)₂ | *37% |
| ****4** | 200 W | 380 µm | 15 µl/min | DMF/H₂O Pd(OAc)₂ | 26% |
| ****5** | 170 W | 380 µm | 15 µl/min | DMF/H₂O Pd(OAc)₂ | 28% |

| | | | | | |
|---|---|---|---|---|---|
| **Scheme 7 conditions:** 1 equiv. of arylhalide, 1.2 equiv. boronic acid, 3 equiv. of base, 5 mol% Pd catalyst in DMF/H₂O. * compound isolated by chromatography ** These reactions were performed with a capillary tube which was coated internally with palladium. | | | | | |

### Example 8 an example that does not specifically embody the claimed invention

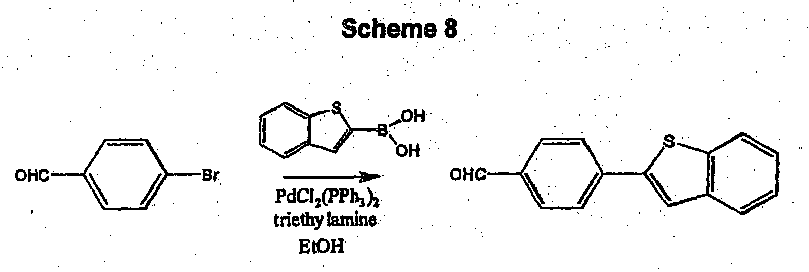

| ***Entry*** | ***Power*** | ***Capillary Diameter*** | ***Flowrate*** | ***Solvent*** | ***Conversion*** |
|---|---|---|---|---|---|
| **1** | 170 W | 380 µm | 25 µl/min | EtOH | 25% |

| | | | | | |
|---|---|---|---|---|---|
| **Scheme 8 conditions:** 1 equiv. of arylhalide, 1 equiv. boronic acid, 3 equiv. of triethylamine, 5 mol% Pd catalyst in EtOH. | | | | | |

### Example 9

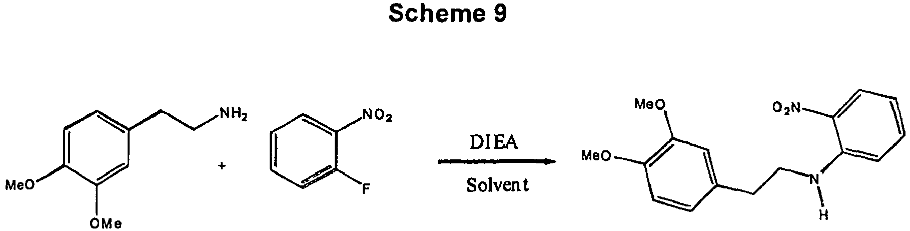

| ***Entry*** | ***Power*** | ***Capillary Diameter*** | ***flowrate*** | ***solvent*** | ***Conversion*** |
|---|---|---|---|---|---|
| 1 | 150 W | 380 µm | 25 µl/min | EtOH | 68% |
| 2 | RT | RBF | Batch Control | EtOH | 0% |
| 3 | 200 W | 380 µm | 10 µl/min | EtOH | 83% |
| 4 | 170 W | 380 µm | 25 µl/min | DMF | 100% |
| 5 | RT | RBF | Batch Control | DMF | 68% |
| 6 | 100 W | *380 µm | 25 µl/min | ETCH | 68% |
| 7 | 150 W | 380 µm | 25 µl/min | **EtOH | 56% |
| 8 | 150 W | 380 µm | 25 µl/min | EtOH | 55% |
| 9 | 200 W | 380 µm | 2-5 µl/min | EtOH | 40% |
| 10 | 170 W | 1100-1200 µm | 10 µl/min | EtOH | 75% |
| 11 | 200 W | 1100-200 µm | 5 µl/min | EtOH | 77% |

| | | | | | |
|---|---|---|---|---|---|
| **Scheme 9 conditions:** 1 mmol of fluoronitrobenzene, 2 mmol of diisopropylethylamine and 2 mmol of 3,4-Dimethoxyphenylethylamine. * This reaction was performed with a capillary tube which was coated internally with palladium ** same conditions as entry 1 with the exception that the concentration of starting reagents diluted by a factor of 2. Entry 2 refers to the identical reaction as entry 1, performed under similar conditions (i.e. same concentration) using "standard" chemical techniques (i.e. round bottomed flask with stirrer) at room temperature. Entry 5 refers to the identical reaction as entry 4, performed under similar conditions (i.e. same concentration) using "standard" chemical techniques (i.e. round bottomed flask with stirrer) at room temperature. | | | | | |

### Example 10 an example that does not specifically embody the claimed invention

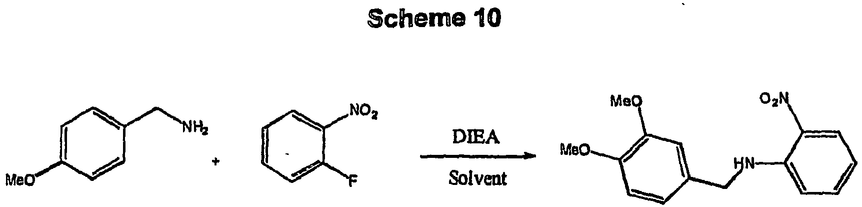

| ***Entry*** | ***Power*** | ***Capillary Diameter*** | ***Flowrate*** | ***Solvent*** | ***Conversion*** |
|---|---|---|---|---|---|
| **1** | 170W | 380 µm | 25 µl/min | EtOH | 84% con |
| **2** | RT | REF | Batch control | EtOH | 13 % con |
| **3** | 170 W | 380 µm | 25 µl/min | DMF | 92% con |
| **4** | RT | RBF | Batch control | DMF | 31 % con |

| | | | | | |
|---|---|---|---|---|---|
| **Scheme 10 conditions:** 1 mmol of fluoronitrobenzene, 2 mmol of diisopropylethylamine and 2 mmol of 4-methoxybenzylamine. Entry 2 refers to the identical reaction as entry 1, performed under similar conditions (i.e. same concentration) using "standard" chemical techniques (i.e. round bottomed flask with stirrer) at room temperature. Entry 4 refers to the identical reaction as entry 3, performed under similar conditions (i.e. same concentration) using "standard" chemical techniques (i.e. round bottomed flask with stirrer) at room temperature. | | | | | |

### Example 11 an example that does not specifically embody the claimed invention

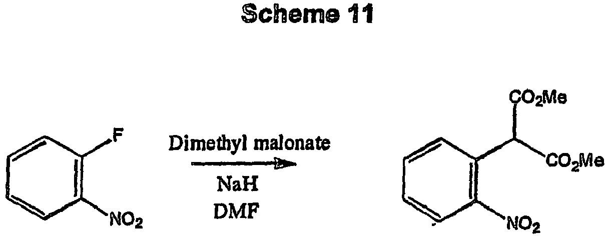

| ***Entry*** | ***Power*** | ***Capillary Diameter*** | ***Flowrate*** | ***Solvent*** | ***Conversion*** |
|---|---|---|---|---|---|
| **1** | 100 W | 380 µm | 40 µl/min | DMF | 72% |
| **2** | RT | RBF | Batch Control. RT | DMF | 28% |

| | | | | | |
|---|---|---|---|---|---|
| **Scheme 11 conditions:** 1 equiv. of arylhalide, 2.5 equiv. of dimethyl methylmalonate, 2.5 equiv. of sodium hydride, in DMF. Entry 2, batch control rxn, refers to the identical reaction as entry 1, performed under similar conditions (i.e. same concentration) using "standard" chemical techniques (i.e. round bottomed flask with stirrer) at room temperature. | | | | | |

### Example 12 an example that does not specifically embody the claimed invention

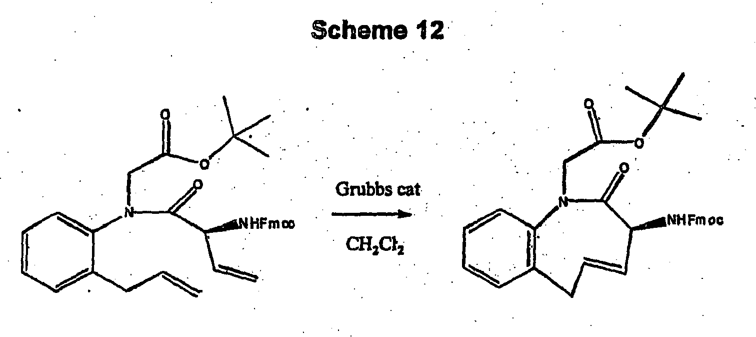

| ***Entry*** | ***Power*** | ***Capillary Diameter*** | ***Flowrate*** | ***Solvent*** | ***Conversion*** |
|---|---|---|---|---|---|
| **1** | 100W | 380 µm | 40 µl/min | CH₂Cl₂ | 100% |

| | | | | | |
|---|---|---|---|---|---|
| **Scheme 12 conditions:** 1 equiv. of diene, 1 mol% Grubbs catalyst in CH₂Cl₂. | | | | | |

### Example 13 an example that does not specifically embody the claimed invention

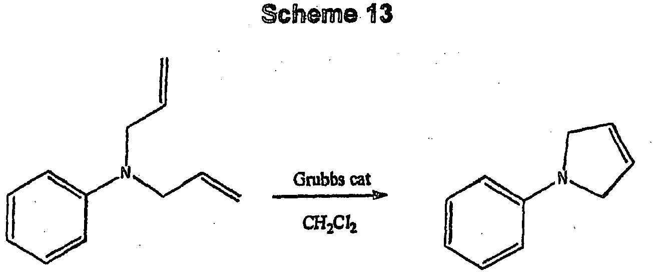

| ***Entry*** | ***Power*** | ***Capillary Diameter*** | ***Flowrate*** | ***Solvent*** | ***Conversion*** |
|---|---|---|---|---|---|
| 1 | 150 W | 380 µm | 30 µl min | CH₂Cl₂ | 45% |

| | | | | | |
|---|---|---|---|---|---|
| **Scheme 13 conditions:** 1 equiv. of diene, 1 mol% Grubbs catalyst in CH₂Cl₂. | | | | | |

### Example 14

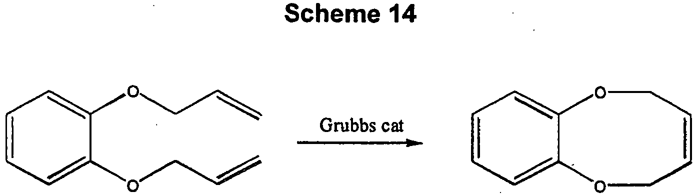

| ***Entry*** | ***Power*** | ***Capillary Diameter*** | ***Flowrate*** | ***Solvent*** | ***Conversion*** |
|---|---|---|---|---|---|
| **1** | 150 W | 380 µm | 30 µl/min | CH₂Cl₂ | 28% |
| ***2** | 50. W | 380 µm | 30 µl/min | CH₂Cl₂ | 14% |
| ***3** | 50 W | 380 µm | 40 µl/min | Toluene | 35% |
| ***4** | 20 W | 380 µm | 40 µl/min | Toluene | 13% |
| **5** | RT | RBF | Batch rxn Reflux 16h | CH₂Cl₂ | 32% |
| **6** | RT | RBF | Batch control | CH₂Cl₂ | 10% |

| | | | | | |
|---|---|---|---|---|---|
| **Scheme 14 conditions:** 1 equiv. of diene, 1 mol % Grubbs catalyst in CH₂Cl₂ or toluene. * These reactions were performed with a capillary tube which was coated internally with palladium. Entry 5 refers to the identical reaction as entry 1, performed under similar conditions (i.e. same concentration) using "standard" chemical techniques (i.e. round bottomed flask with stirrer) performed at reflux with the aid of an oil bath. Entry 6 refers to the identical reaction as entry 1, performed under similar conditions (i.e. same concentration) using "standard" chemical techniques (i.e. round bottomed flask with stirrer) at room temperature. **Green Chemistry; Reactions in the section used only water as solvent.** | | | | | |

### Example 15 an example that does not specifically embody the claimed invention

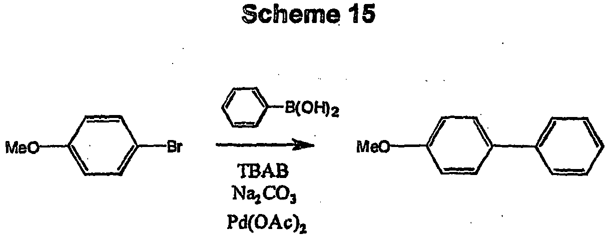

| ***Entry*** | ***Power*** | ***Capillary Diameter*** | ***Flowrate*** | ***Solvent*** | ***Conversion*** |
|---|---|---|---|---|---|
| 1 | 100 W | 380 µm | 25 µl/min | H₂O | 62% |
| 2 | 170 W | 380 µm | 25 µl/min | H₂O | 100% |
| 3 | RT | RBF | Batch Control RT | H₂O | 38% after 1h |

| | | | | | |
|---|---|---|---|---|---|
| **Scheme 15 conditions:** 1 equiv. of arylhalide, 1 equiv. boronic acid, 1 equiv. tetrabutylammonium bromide, 3 equiv. of base 5 mol% Pd catalyst in H₂O. Entry 3, batch control reaction refers to the identical reaction as entry 1, performed under similar conditions (i.e. same concentration) using "standard" chemical techniques (i.e. round bottomed flask with stirrer) performed at room temperature for 1 h. | | | | | |

### Example 16 an example that does not specifically embody the claimed invention

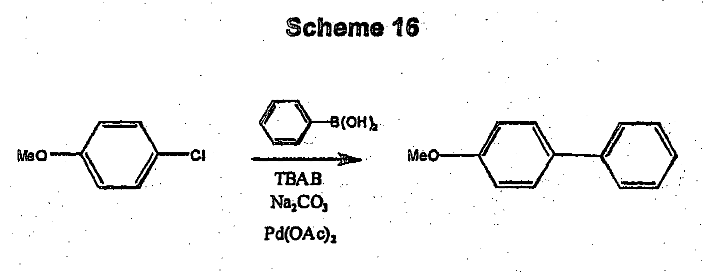

| ***Entry*** | ***Power*** | ***Capillary Diameter*** | ***Flowrate*** | ***Solvent*** | ***Conversion*** |
|---|---|---|---|---|---|
| 1 | 170 W | 380 µm | 25 µl/min | H₂O | 100% |

| | | | | | |
|---|---|---|---|---|---|
| **Scheme 16 conditions:** 1 equiv. of arylhalide, 1 equiv. boronic acid, 1 equiv. tetrabutylammonium bromide, 3 equiv of base, 5 mol% Pd catalyst in H₂O. **Using Two Inlet Streams: reactions in this section used 2 inlet streams each containing a reagent** | | | | | |

### Example 17 an example that does not specifically embody the claimed invention

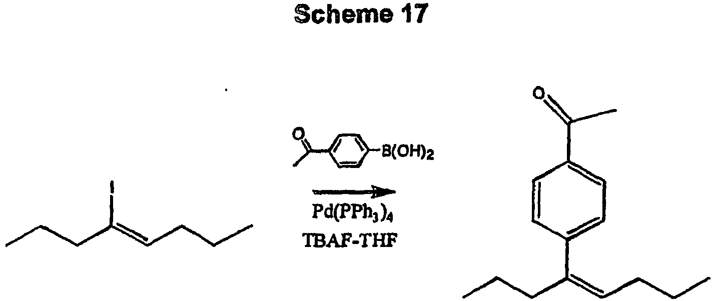

| **Entry** | **Power** | **Capillary Diameter** | **flowrate** | **Solvent** | **Conversion** |
|---|---|---|---|---|---|
| **1** | 100 W | 380 µm | 15 µl/min. | THF | 100% |

| | | | | | |
|---|---|---|---|---|---|
| **Scheme 17 conditions:** Stream A; 1 equiv. of vinylhalide, 5 equiv. of base, 5 mol% Pd(PPh₃)₄ catalyst in THF. Stream B; 1 equiv. boronic acid in THF. | | | | | |

### Example 18

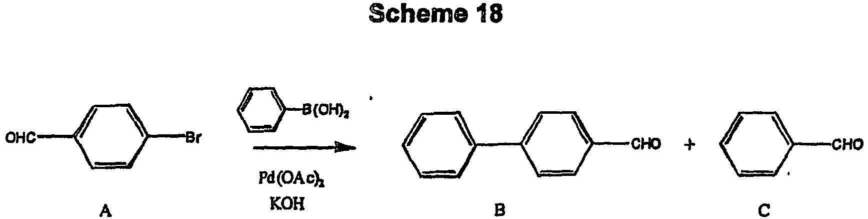

| **Entry** | **Power** | **Capillary Diameter** | **flowrate** | **Solvent** | **Ratio A:B:C** |
|---|---|---|---|---|---|
| ***1** | 100 W | 380 µm | 15 µl/min. | DMF/H₂O | 36(**A**):17(**B**): 47(**C**). |
| **2** | 60 °C | RBF | Batch Reaction | THF | At 1.5h; 29(**A**): 71(**B**). |
| | | | | | At 4 h; 22(**A**): 78 (**B**). |

| | | | | | |
|---|---|---|---|---|---|
| **Scheme 18 conditions:** Stream A; 1 equiv of arylhalide; 5 equiv of base, 5 mol% Pd(OAc)₂ in THF. Stream B; 1.2 equiv boronic acid. Ratio A:B:C represents the ratio of the 2 products B and C relative to starting material A as determined by ¹H NMR. In entries were C is not specified, it was not observed. * This reaction was performed with a capillary tube which was coated internally with palladium. Entry 2, batch reaction refers to the identical reaction as entry 1, performed under similar conditions (i.e. same concentration) using "standard" chemical techniques (i.e. round bottomed flask with stirrer) performed at 60°C with the aid of an oil bath | | | | | |

### Example 19 an example that does not specifically embody the claimed invention

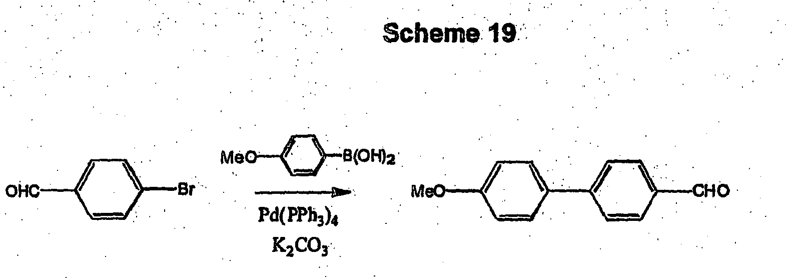

| ***Entry*** | ***Power*** | ***Capillary Diameter*** | ***Flowrate*** | ***Solvent*** | ***Conversion*** |
|---|---|---|---|---|---|
| **1** | 170 W | 380 µm | 25 µl/min | DMF/H₂O | 92% |

| | | | | | |
|---|---|---|---|---|---|
| **Scheme 19 conditions:** Stream A; 1 equiv of arylhalide, 5 equiv of base, 5 mol % Pd catalyst in THF. Stream B; 1.2 equiv boronic acid. | | | | | |

### Example 20 an example that does not specifically embody the claimed invention

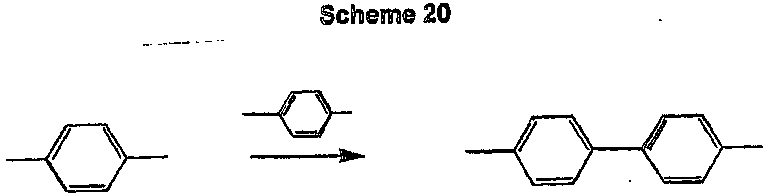

| ***Entry*** | ***Power*** | ***Capillary Diameter*** | ***Flowrate*** | ***Solvent*** | ***Conversion*** |
|---|---|---|---|---|---|
| **1** | 170W | 380 µm | 25 µl/min | DMF/H₂O | 25% |
| **2** | 170W | 380 µm | 25 µl/min | DMF/H₂O | 39% |

| | | | | | |
|---|---|---|---|---|---|
| **Scheme 20 conditions:** Stream A; 1 equiv. of arylhalide, 5 equiv. of base 5 mol% Pd catalyst in THF. Stream B; 1.2 equiv. boronic acid. | | | | | |

### Example 21 an example that does not specifically embody the claimed invention

### Example 21a : Suzuki-Miyaura coupling an example that does not specifically embody the claimed invention

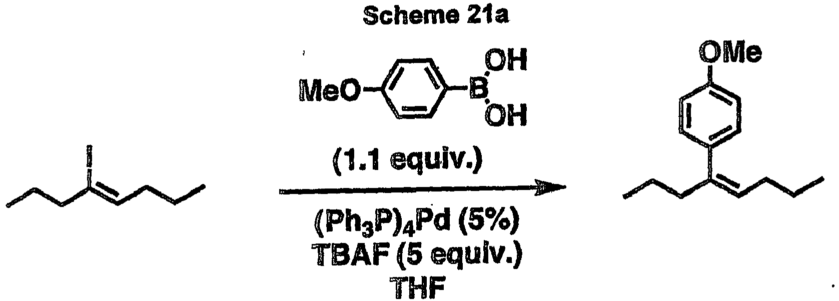

| Entry | Time | Power | Capillary Diameter | Flowrate (µL/min) | Conversion |
|---|---|---|---|---|---|
| 1 | 60 min | RT | RBF | Control | 0 % |
| 2 | 15 min | 100 W | 200 µm | No flow sealed tube | 100 % |
| 3 | 13.9 min | 100W | 200 µm | 2 | 100 % |

Scheme 21 a shows a Suzuki-Miyaura coupling reaction. Entry 1 represents the reaction carried out under standard conditions in a RBF at RT. Entry 2 represents the same reaction carried out in a capillary under 100 W microwave irradiation in a sealed tube. Entry 3 represents the reaction carried out under similar conditions to entry 2 with premixed solutions flowed though one inlet. Both microwave reactions gave full conversion to the desired final product, while the standard method gave no conversion to the desired product.

### Example 21b: Ring-closing metathesis an example that does not specifically embody the claimed invention

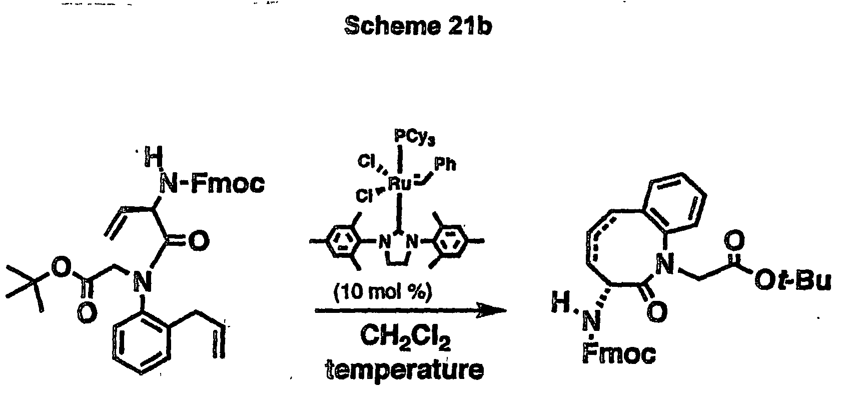

| Entry | Time | Power | Capillary Diameter | Flowrate (µL/min) | Conversion |
|---|---|---|---|---|---|
| 1 | 30 min | 35 °C | RBF | Control | 30 % |
| 2 | 30 min | 200 W | Microwave vial (std) | Control | 100 % |
| 3 | 1.9 min | 100 W | 380 µm capillary | 40 | 100 % |

Scheme 21b shows a ring-closing metathesis reaction. As in Example 21a, Entry 1 represents the experiment under standard conditions, Entry 2 under microwave conditions with no flow (in this case in a microwave vial), and 3 under microwave conditions with flow though one inlet. Again conversion to the final product was 100% under both microwave conditions while the standard reaction conditions yielded only 30% conversion to product.

### Example 21 c: Wittig olefination an example that does not specifically embody the claimed invention

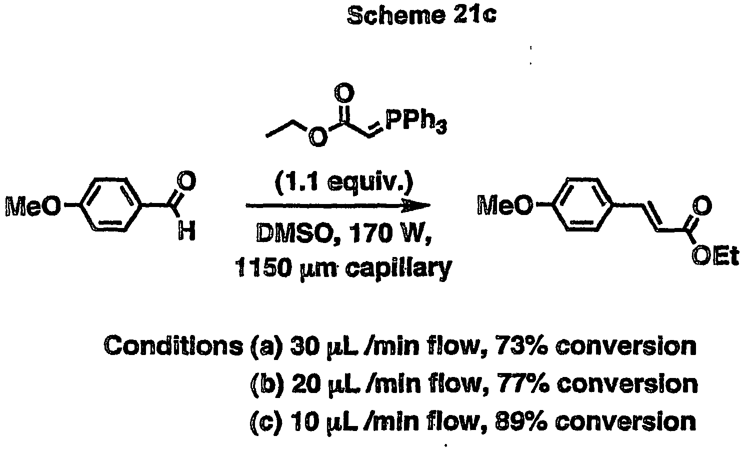

| Entry | Power | Capillary Diameter | Flowrate (µL/min) | Conversion |
|---|---|---|---|---|
| 1 | 170W | 1150 µm capillary | 30 | 73 % |
| 2 | 170 W | 1150 µm capillary | 20 | 77 % |
| 3 | 170 W | 1150 µm capillary | 10 | 89 % |
| 4* | 280 W | | No flow sealed tube | 54% |

Scheme 21 c shows a Wittig olefination reaction. This experiment demonstrates the effect of flow rate on the reaction kinetics. In this case a slower flow rate resulted in greater yield of products however even the faster flow rate gave improved yield over no flow. * Entry 4 is based on results reported in available literature. The reaction was fully heterogeneous. Such conditions would likely pose a serious problem for prior art microchannel reactor technology as it would lead to clogged channels and/or frits.

### Example 22: Examining reaction parameters

### Example 22a: Power Setting an example that does not specifically embody the claimed invention

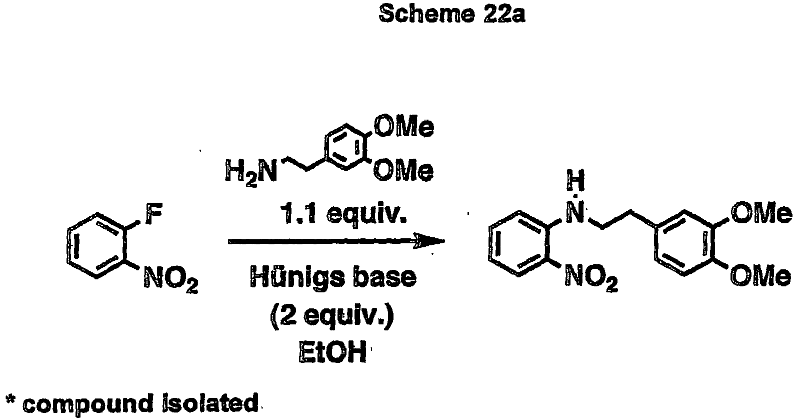

### Power Setting

| **Entry** | **Power** | **Capillary Diameter** | **Flowrate** | **Molarity (M)** | **Conversion** |
|---|---|---|---|---|---|
| 1 | 200 W | 380 µm | 30 µL min | 0.43 | 61% |
| 2 | 100 W | 380 µm | 30 µL min | 0.43 | 58% |
| 3 | 50 W | 380 µm | 30 µL min | 0.43 | 41% |

Scheme 22a shows a nucleophilic aromatic substitution reaction. This Example demonstrates the effect of varying the power setting on such reactions. The reaction conditions were not optimized as they were designed to show relative differences. While the higher power setting often resulted in better yields, this was not always the case. Higher temperatures can result in decomposition of the catalysts, lowering yield.

### Example 22b: Capillary Diameter an example that does not specifically embody the claimed invention

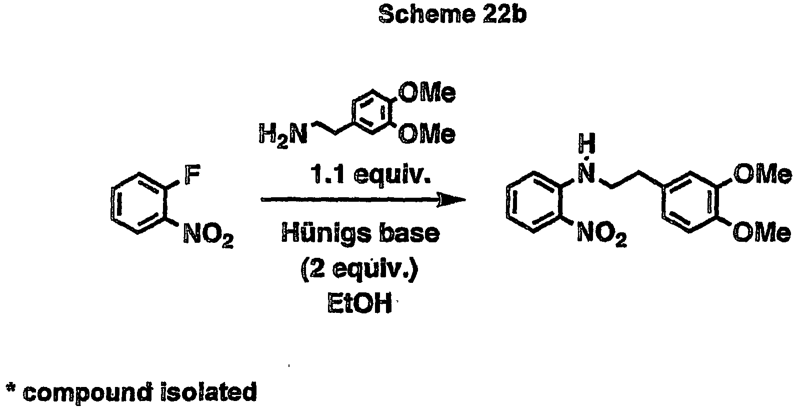

### Capillary Diameter

| **Entry** | **Power** | **Capillary Diameter** | **Flowrate** | **Molarity (M)** | **Conversion** |
|---|---|---|---|---|---|
| 1 | 150 W | 200 µm | 30 µL min | 0.43 | 47% |
| 2 | 150 W | 326 µm | 30 µL min | 0.43 | 55% |
| 3 | 150 W | 380 µm | 30 µL min | 0.43 | 60% |
| 4 | 150 W | 1100-1200 µm | 30 µL min | 0.43 | 57% |

Scheme 22b shows a nucleophilic aromatic substitution reaction. This Example demonstrates the effect of varying the capillary diameter on such a reaction. The reaction conditions were not optimized as they were designed to show relative differences. It is apparent that a larger capillary diameter yields improved conversion but that a certain size, further increasing the capillary diameter, can produce a lower yield.

### Example 22c: Flowrate an example that does not specifically embody the claimed invention

| **Entry** | **Power** | **Capillary Diameter** | **Flowrate** | **Molarity (M)** | **Conversion** |
|---|---|---|---|---|---|
| 1 | 154 W | 380 µm | 15µL min | 0.43 | 76% |
| 2 | 150 W | 380 µm | 45 µL min | 0.43 | 57% |
| 3 | 150 W | 380 µm | 60 µL min | 0.43 | 54% |

The reaction presented in Scheme 22b was repeated with varying flowrates. The reaction conditions were not optimized as they were designed to show relative differences. This experiment demonstrates that the slower the flow rate, the better the yield.

### Example 22d: Reaction Concentration an example that does not specifically embody the claimed invention

The reaction presented in Scheme 22b was repeated with varying reactant concentrations.

| **Entry** | **Power** | **Capillary Diameter** | **flowrate** | **Molarity (M)** | **Conversion** |
|---|---|---|---|---|---|
| 1 | 150 W | 380 µm | 30 µL min | 0.23 | 52% |
| 2 | 150 W | 380 µm | 30 µL min | 0.43 | 60% |
| 3 | 150 W | 380 µm | 30 µL min | 0.74 | 66% |

The reaction conditions were not optimized as they are designed to show relative differences. Generally it was found that reactions hollowed standard rules of kinetics, i.e., the higher the concentration, the faster the rate.

### Example 22e: Effects of Coating Capillary with Thin Metal Film

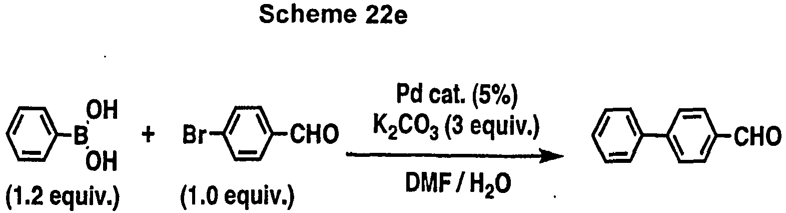

| Conditions | Power (W) | Flowrate (mL/min) | Capillay diameter (µm) | Percent Conversion |
|---|---|---|---|---|
| Pd(OAc)₂ K₂CO₃ DMF/H₂O | 170 | 30 | 1150 | 38 % (62% recovered) |
| Pd(OAc)₂ KOH DMF/H₂O | 170 | 30 | 1150 | 100 % Pd Precipitated to coat capillaries |

Scheme 22e shows a Suzuki-Miyaura coupling reaction performed in capillaries that were not coated with metal. Pd metal blacked out during the reactions with KOH. In the KOH run, the palladium catalyst provided in the solution started to precipitate as a coating or film during the reaction.

### Example 22f: Effects of coating capillary with thin metal film: No Pd Catalyst added

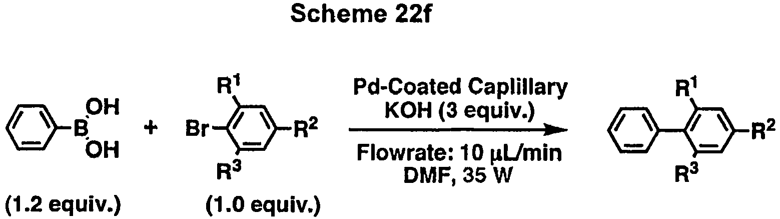

| Substituents | Product | Conversion |
|---|---|---|
| R¹ = R³ = H, R² = CHO | 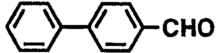 | 89 % |
| R¹ = R³ = H, R² = CH₃ | 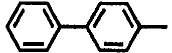 | 95 % |
| R¹ = R³ = H, R² = OCH₃ | 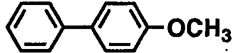 | 97 % |
| R¹ = R³ = R² = CH₃ | 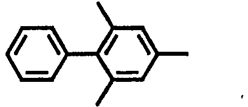 | 92% |

Scheme 22f shows a Suzuki-Miyaura coupling reaction with reactants having different substituents and with metal coated capillaries (i.e. capillaries 112 with lining 125). It was found that the metal lining dramatically increased reaction temperature and also percent conversion. The metal thin film itself can catalyse coupling reactions and no additional metal catalyst need be added. Using the metal-coated capillaries, much lower power settings were sufficient to produce very high temperatures at the reaction site.

### Example 22g: Effects on Reactions with Very High Reaction Barriers Diels Alder cycloaddition

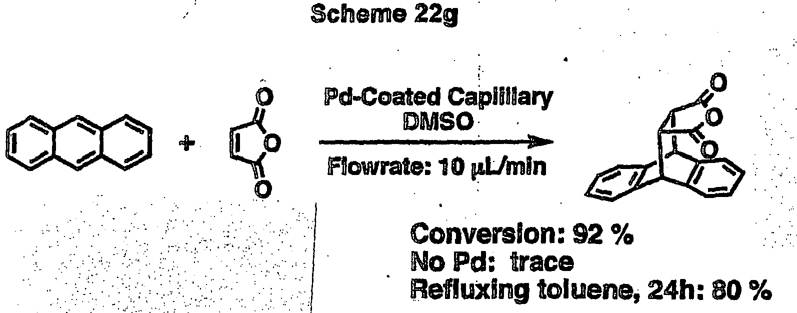

Scheme 22g shows a Diels Alder cycloaddition reaction performed, in a Pud-coated capillary. This experiment demonstrates that metal-coated capillaries can be used with microwave irradiation to achieve good conversions for reactions with a very high reaction barrier, It was found that irradiations of the metal-coated capillary alone, with no solvent can produce steady temperatures of up to 300°C.

### Example 23: Experiments to improve reaction throughput. an example that does not specifically embody the claimed invention

### Example 23a: Two, Inlets from Two Syringes. an example that does not specifically embody the claimed invention

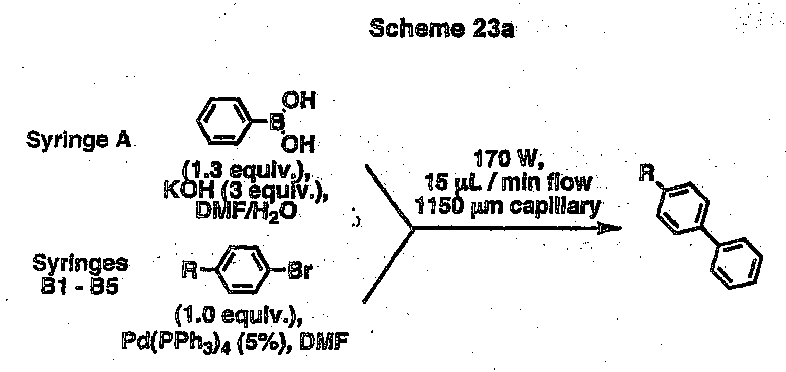

| **Bromide (B1-3)** | Product | Yield | Bromide (B4-5) | Product | Yield |
|---|---|---|---|---|---|
| 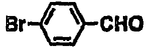 | 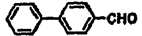 | 97 % | 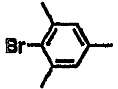 | 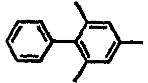 | 72 % |
| 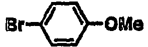 | 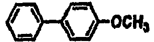 | 96 % | 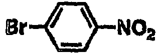 | 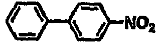 | 100 % |
| 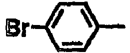 | 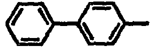 | 90% | | | |

Scheme 23a shows a Suzuki-Miyaura coupling reaction performed using two inlets from two syringes to introduce reagents into the reaction mixture. This process may be referred to as "mixing on the fly". The experiment demonstrates that the reaction can be carried out on substrates with a variety of substituents. The percent conversion varied depending on the substrates used in the reaction.

### Example 23b: Parallel Capillary Irradiation, Using a Multi-Inlet Reactor an example that does not specifically embody the claimed invention

This example was performed using an apparatus similar to that of Figure 4 and 6. "Syringe A1" referred to below generally equates to a supply of reagent 230a; "Syringe A2" to reagent 230b; "Syringe B1" to reagent 230c; and "Syringe B2" to reagent 230d.

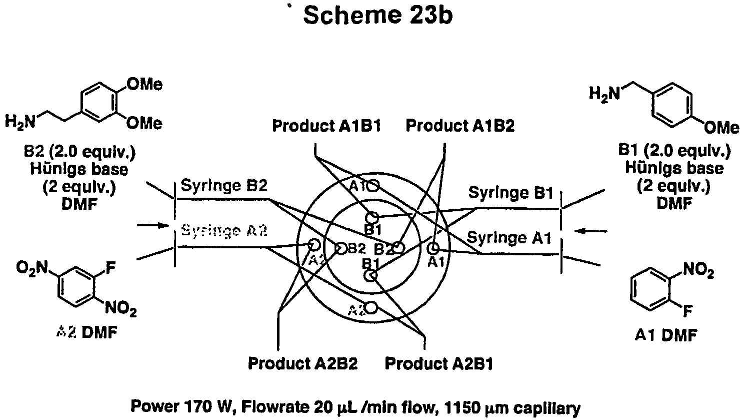

| | | |
|---|---|---|
| | Fluoride A1 | Fluoride A2 |
| | 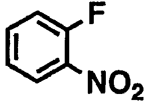 | 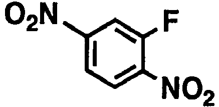 |
| Amine B1 | Product A1B1 (78 %) | Product A2B1 (100 %) |
| 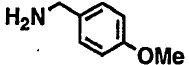 | 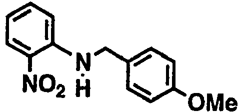 | 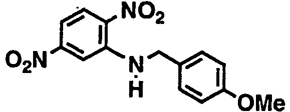 |
| Amine B2 | Product A1B2 (90 %) | Product A2B2 (100 %) |
| 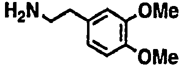 | 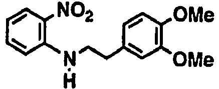 | 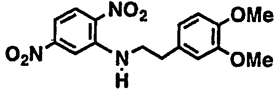 |

Scheme 23b shows a nucleophilic aromatic substitution performed in parallel, using multi-inlet reactor similar to reactor 210 of Figure 4. This method was used to prepare libraries of compounds by continuous flow, simultaneous, parallel, capillary irradiation, using a multi-inlet reactor. This example shows the preparation of a collection or library of secondary amines prepared by nucleophilic aromatic substitution. The library was prepared by continuous flow, simultaneous, parallel capillary irradiation using a multi-inlet reactor. The following conditions were used for all of the experiments: 1 equiv. of reagents A in DMF, 2 equiv. of B in DMF 170 W, 1150 mm capillary, 20 mL min. The simultaneous parallel capillary experiment yielded good conversions, no interference was observed due to the presence of several capillaries in the chamber at once.

### Example 23c: Simultaneous Sequential Parallel Capillary Irradiation an example that does not specifically embody the claimed invention

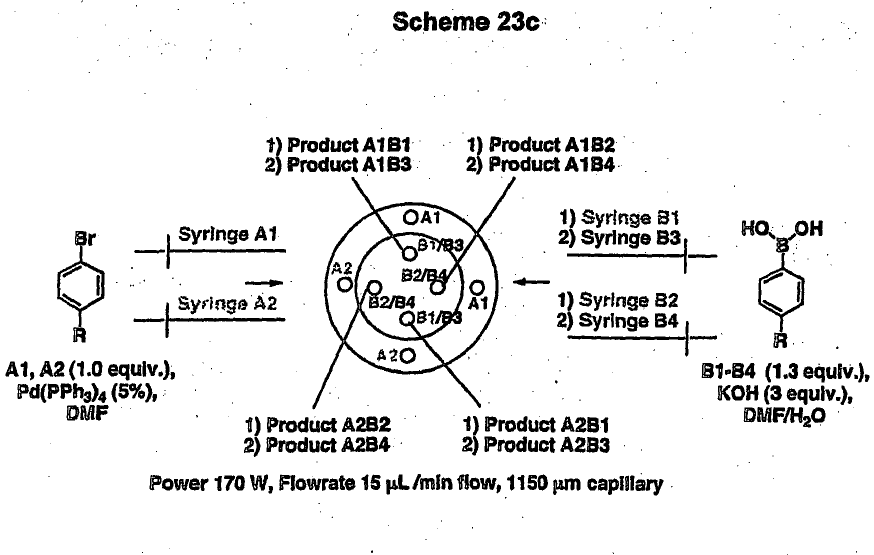

| | | |
|---|---|---|
| | Bromide A1 | Bromide A2 |
| Boronic Acid | 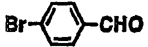 | 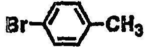 |
| B1 | A1B1 (100%) | A2B1 (67%) |
| 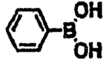 | 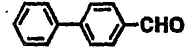 | 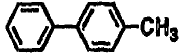 |
| B2 | A1B2 (100 %) | A2B2 (91%) |
| 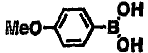 | 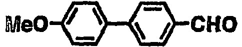 | 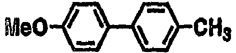 |

| Run 1: 6 minutes, Switch valve to rinse, Switch valve to new Boronic acid | | |
|---|---|---|
| B3 | A1B3 (100%) | A2B3 (92%) |
| 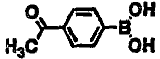 | 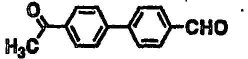 | 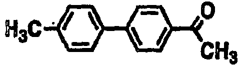 |
| B4 | A1B4 (100%) | A2B4 (91 %) |
| 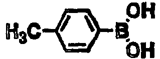 | 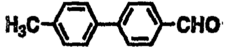 | 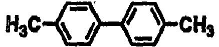 |

Scheme 23c shows the preparation of libraries of compounds by a cross coupling reaction using *continuous flow, simultaneous, sequential, parallel* capillary irradiation using a multi-inlet reactor system. The substrates can be switched and infused through the parallel reactor to prepare compounds that are separated in time as shown in the scheme above. The reactions conditions were as follows: (Ph₃P)₄Pd (5%), K₂CO₃ (5 equiv.), DMF / H₂O

### Example 23d: Multi-Component Reactions Using Continuous Flow, Sequential, Capillary Irradiation in a Multi-inlet Reactor an example that does not specifically embody the claimed invention

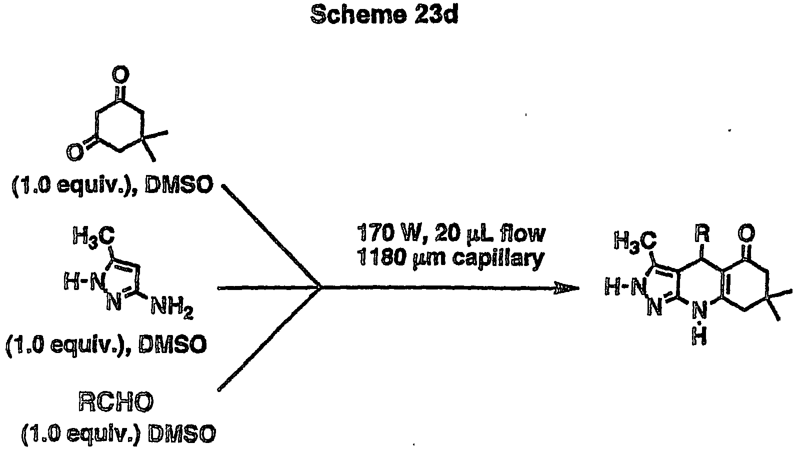

Scheme 23d shows a 3-component reaction and this experiment demonstrates the use of the multi-inlet reactor in the preparation of compounds from multi-component reactions.

### Example 23e: Multi-Component Reactions Using Continuous Flow, Sequential, Capillary Irradiation in a Multi-inlet Reactor an example that does not specifically embody the claimed invention

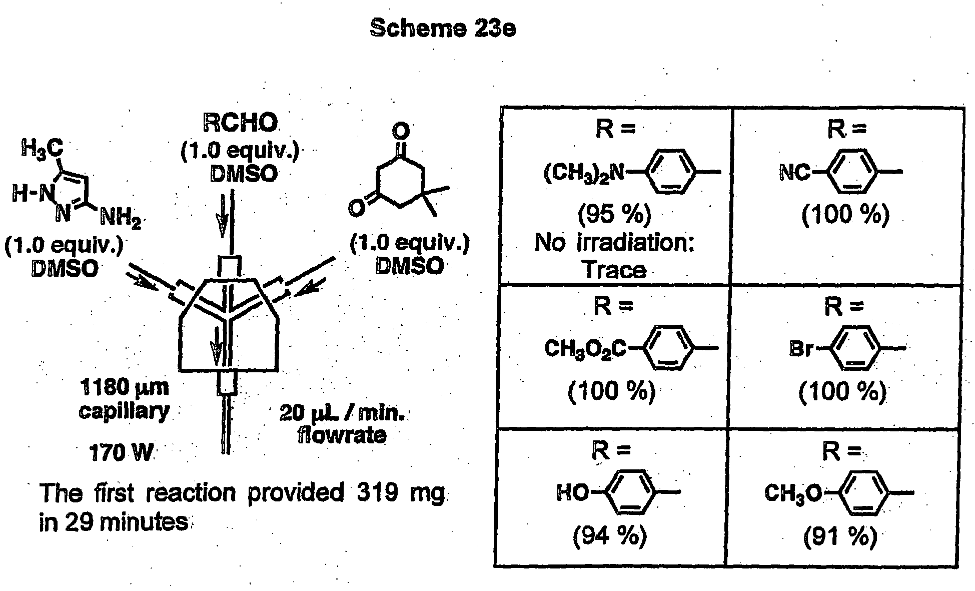

Scheme 23e shows a reaction involving a 3-component library This example demonstrates the use of the multi-inlet reactor for the preparation of libraries of compounds made by continuous flow, sequential; parallel, 3-component reaction.

### Example 23f: Additional, Multi-Component Reaction Using Continuous (Flow, Sequential, Capillary Irradiation in a Multi-inlet Reactor an example that does not specifically embody the claimed invention

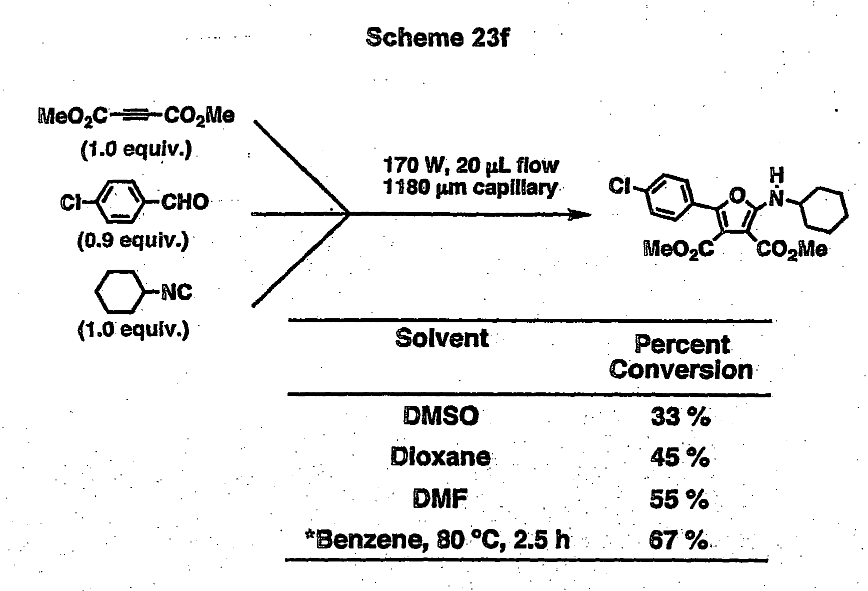

*The percent conversion in Benzene was determined from the literature.
Scheme 23f shows a 3-component cyclization reaction to provide furans made by continuous flow, sequential, parallel, 3-component reactions;

### Example 23g: Additional Multi-Component Reaction Using Continuous flow, Sequential; Capillary Irradiation in a Multi-inlet Reactor an example that does not specifically embody the claimed invention

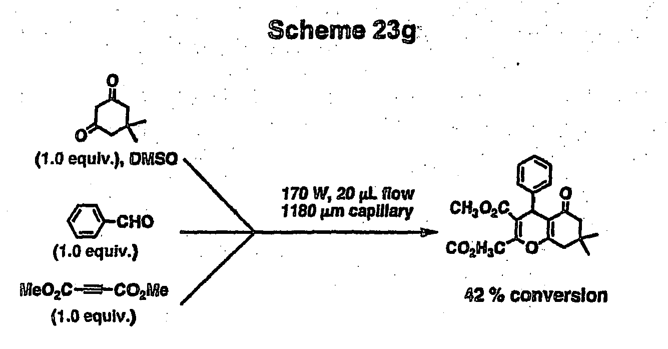

Scheme 23g shows a 3-component cyclization reaction to provide fused pyrans furans made by continuous flow, sequential, parallel, 3-component reactions

### Example 23h: Multi-Component, Multi-Step, Reactions Using Continuous Flow, Sequential, Capillary Irradiation in a Multi-inlet Reactor an example that does not specifically embody the claimed invention

Not all functional groups are compatible for 'all-in-one' multi-components reactions. The parallel reactor was adapted for 'queued' reactions where reaction intermediates, rather than starting materials, can be flowed through it or back through it. This reactor is shown schematically in Figure 8.

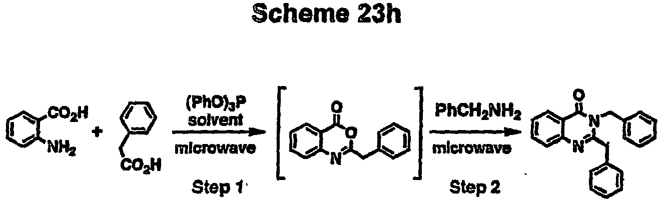

| Step | Capillary | Solvent | Flowrate | Conversion |
|---|---|---|---|---|
| 1 | 1180 µm | pyridine | 30 | 0 % |
| 1 | 1180 µm | pyridine | 60 | 3 % |
| 1 | 1180 µm - Pd | pyridine | 30 | 82 % |
| 1 | 1180 µm | DMF | 30 | 0 % |
| 1 | 1180 µm - Pd | DMF | 60 | 0% |
| 2 | 1180 µm - Pd | pyridine | 30 | complete |

Scheme 23h shows a 3-component reaction to provide quinazolinones. This scheme shows multi-component, multi-step experiments conducted under various conditions. White optimization of the conditions may be required it is clear that complex reactions of this type can be carried out using microwave irradiation in a multi-inlet reactor.

### Continuous Flow Library

**Table 1 an example that does not specifically embody the claimed invention**

| | | | | |
|---|---|---|---|---|
| **Compound 1** | 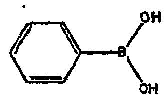 | **Product** | **Conditions** | **Conversion** |
| **Compound 2-1** | 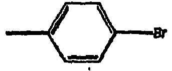 | 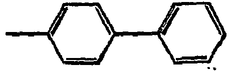 | 25 µl min 100W 380 µm capillary | **100%** |
| **Compound 2-2** | 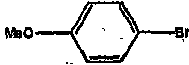 | 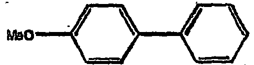 | 25 µl min 100 W 380 µm capillary | ***96%** |
| **Compound 2-3** | 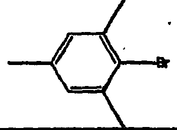 | 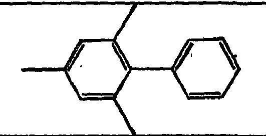 | 25 µl min 100W 380 µm capillary | ***19%** |
| **Compound 2-4** | 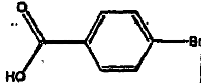 | 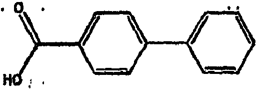 | 25 µl min 100 w 380 µm capillary | ***100%** |
| **Compound 2-5** | 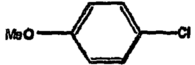 | 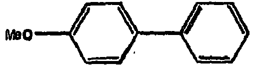 | 25 µl min 100 W 380 µm capillary | **Trace Quantities (1%)** |

| | | | | |
|---|---|---|---|---|
| *** compound isolated** **Table 1 condition:** 1 mmol of arylhalide, 1 mmol of boronic acid, 3 mmol of NaS₂CO₃, 5 mol% Pd(OAc)₂, 1 mmol of tetrabutylammonium bromide, 2 ml of water. | | | | |

The above details a library produced continuously. This library can be performed using 1 inlet stream in which the reagents are premixed and flowed through the system or via 2 inlet streams in which compound 1 is flowed continuously through inlet 1 while compounds 2 are introduced via inlet 2.

**Table 2 an example that does not specifically embody the claimed invention**

| | | | | |
|---|---|---|---|---|
| **Stream A** | 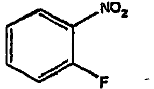 | **Product** | **Conditions** | **Conversion** |
| **Stream B Compound 1** | 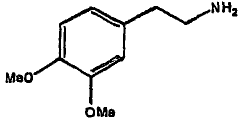 | 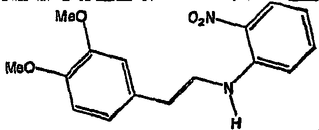 | 30 µl min 180W 380 µm capillary | **87%** |
| **Stream B Compound 2** | 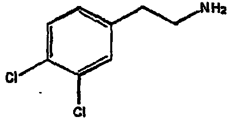 | 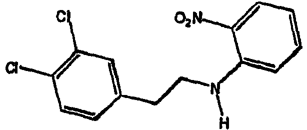 | 30 µl min 180W 380 µm capillary | ***57%** |
| **Stream B Compound 3** | 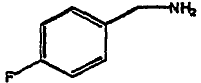 | 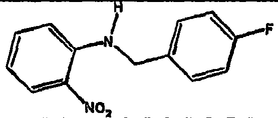 | 30 µl min 180W 380 µm capillary | ***64%** |
| **Stream B Compound 4** | 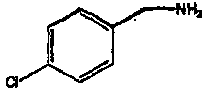 | 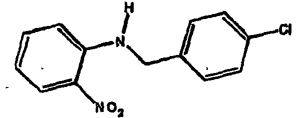 | 30 µl min 180W 380 µm capillary | ***47%** |
| **Stream B Compound 5** | 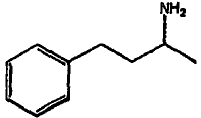 | 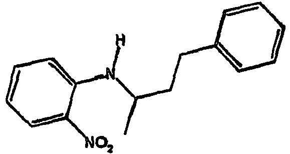 | 30 µl min 180 W 380 µm capillary | ***40%** |

| | | | | |
|---|---|---|---|---|
| **Table 2 conditions**: Stream A 1 mmol of fluoronitrobenzene and 2 mmol of diisopropylethylamine in DMF. Stream B 1 mmol of amino compounds 1-5 in DMF *** compound isolated** | | | | |

The above details a library produced continuously. This library can be performed using 1 inlet stream in which the reagents are premixed and flowed through the system or via 2 inlet streams in which stream A contains the fluoronitrobenzene and base while stream B contains the substrate amine.

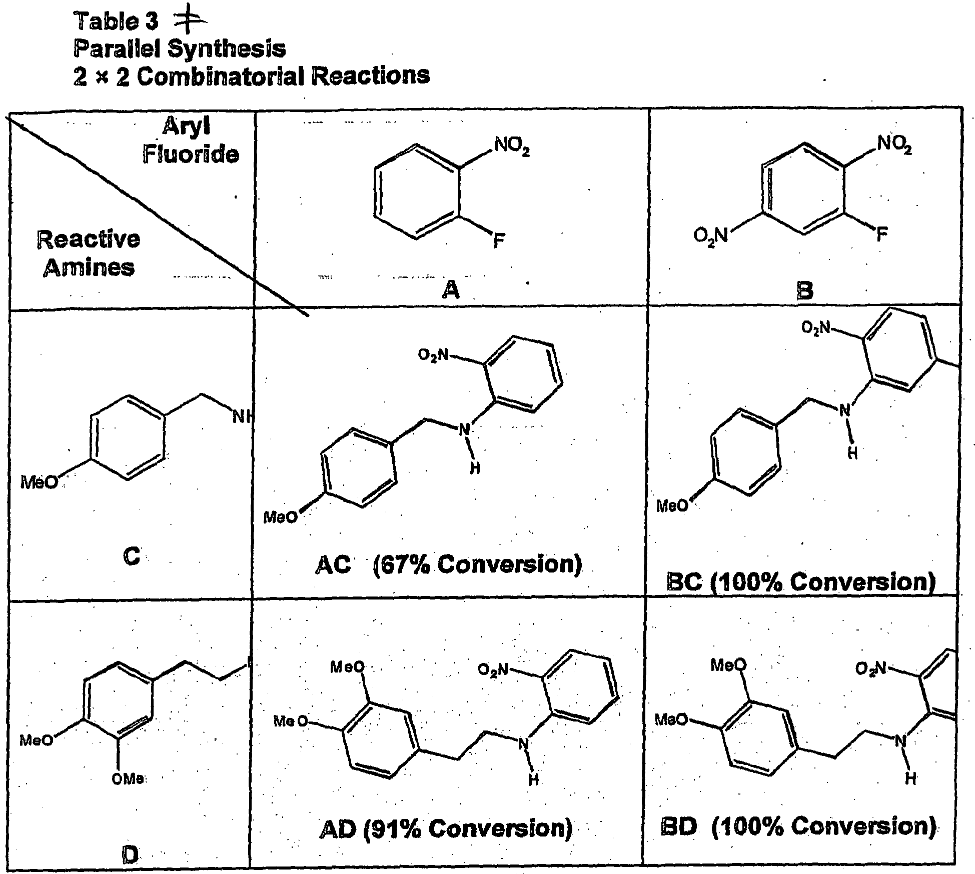

**Conditions:** The apparatus used is similar to that shown in Figure 4.

Syringe A; 1 mmol of 1-fluoro-2-nitrobenzene in 1 ml of DMF.
Syringe B; 1 mmol of 1-fluoro-2,4-dinitrobenzene in 1 ml of DMF.
Syringe C; 2 mmol of 4-methoxybenzylamine and 2 mmol of Diisopropylethylamine in 1 ml of DMF.
Syringe D; 2 mmol of 2-(3,4-Dimethoxy-phenyl-ethylamine and 2 mmol of Diisopropylethylamine in 1 ml of DMF.

With reference also to Figure 6,
Inlets A and C merge to a single channel to produce outlet AC. (240a)
Inlets A and D merge to a single channel to produce outlet AD. (240c)
Inlets B and C merge to a single channel to produce outlet BC. (240d)
Inlets B and D merge to a single channel to produce outlet BD. (240b)

Syringes A, B, C and D were set up as shown in Figure 3. The solutions were passed through the system at 20 µl/min using a syringe pump. Microwave heating was performed at power level of 170 W.

**Table 4**

| | | | | | | |
|---|---|---|---|---|---|---|
| Suzuki-Miyara coupling of aryl boronic acids and bromides using MACOS through Pd-coated capillaries. (MACOS = Microwave Assisted Continuous Organic Synthesis) | | | | | | |
| 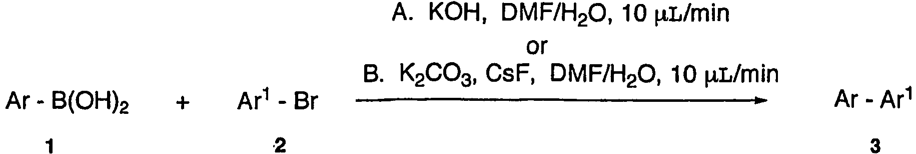 | | | | | | |

| **Entry** | **Ar' (1)** | **Ar" (2)** | **Cond.^{a}** | **T^{b} (°C)** | **Product (2)** | **Convert^{d} (yield)** |
|---|---|---|---|---|---|---|
| 1 | 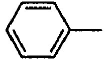 | 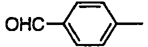 | A | 205 | 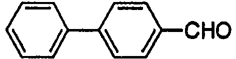 | 88% |
| 2 | 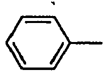 | 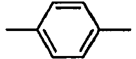 | A | 200 | 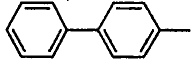 | 95% |
| 3 | 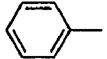 | 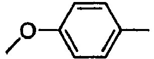 | A | 205 | 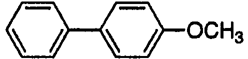 | 96.5% |
| 4 | 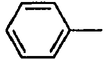 | 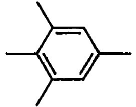 | A | 200 | 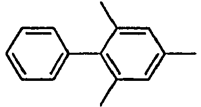 | 92% |
| 5 | 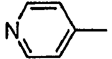 | 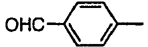 | B | 215 | 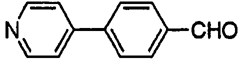 | 93% |
| 6^{f} | 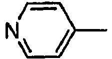 | 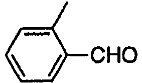 | B | 220 | 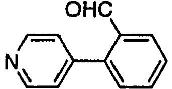 | 59% |
| 7^{f} | 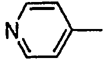 | 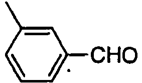 | B | 210 | 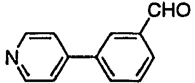 | 73% |
| 8 | 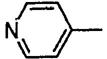 | 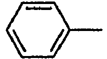 | B | 215 | 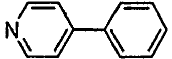 | 81% (74%)^{e} |
| 9^{f} | 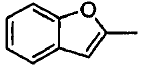 | 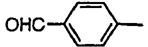 | B | 225 | 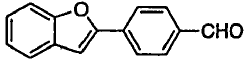 | 76% (84%)^{e} |

| | | | | | | |
|---|---|---|---|---|---|---|
| ^{a}Reaction solutions were flowed through the Pd-coated capillary via a single inlet while being irradiated (see Fig. 1). ^{b}Refers to the temperature on the outer surface of the Pd-coated capillary as measured by the IR sensor of the Smith Creator microwave. ^{c}All reactions were performed using a 1150 micron (ID) Pd coated capillary. ^{d}Percent conversion was determined by withdrawing a crude sample directly from the effluent from the capillary and analyzing it by ¹H NMR spectroscopy. The ratio of starting material to product determined the percent conversion (there were no visible byproducts present, only starting material and product were present in all cases). ^{e}Isolated yield was determined by capturing a known volume of effluent from the capillary and purifying the product by silica gel chromatography. From the volume, the actual amount (mmol) of starting material could be calculated. ^{f}In this case, 2.0 equivalents aryl boronic acid were used. | | | | | | |

**Table 5**

| |
|---|
| **Heck coupling of aryl iodides with acrolein derivatives using MACOS through Pd coated capillaries.** |

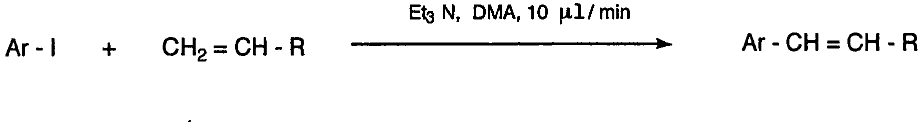

| **Entry** | **Ar^{a}** | **R** | **T^{b} (°C)** | **Product** | **Conversion (Yield)** |
|---|---|---|---|---|---|
| 1 | 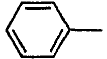 | -CO₂CH₃ | 205 | 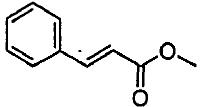 | 80% |
| 2 | 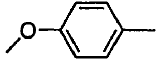 | -CO₂CH₃ | 200 | 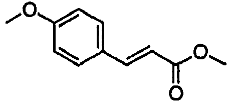 | 58% |
| 3 | 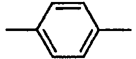 | -CO₂CH₃ | 205 | 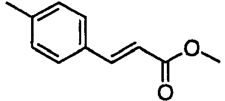 | 88.5% |
| 4 | 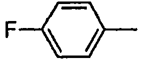 | -CO₂CH₃ | 200 | 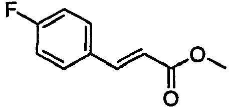 | 88.5% (79%)^{d} |
| 5 | 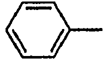 | -CO₂C(CH₃)₃ | 215 | 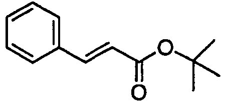 | 99% |
| 6 | 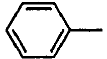 | CN | 220 | 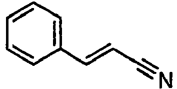 | 99% (82%)^{d} |

| | | | | | |
|---|---|---|---|---|---|
| ^{a} Reaction solutions containing aryl iodide (1.0 equiv.), acrylate (1.3 equiv.) and base (3.0 equiv.) in solvent were premixed and flown through the Pd coated microcapillary via a single inlet while being irradiated at the specified flowrate ^{b} Refers to the temperature on the outer surface of the Pd coated microcapillary as measured by the IR sensor. Percent conversion was determined by ¹H NMR spectroscopy relative to the residual starting material. ^{d} Isolated yield was determined from the product isolated from the small volume of sample collected using the above procedure. | | | | | |

## Claims

1. A reactor apparatus (110,210) comprising:
a) at least one reaction capillary (112,212) having a lumen (126) for receiving one or more reactants (116,216) to undergo a reaction;
b) a magnetron (132,232) for irradiating the one or more reactants (116,216) contained in at least a portion of the capillary (112,212) with microwaves; and
c) a film lining (125) provided on an inner surface (121) of the capillary (112,212) for facilitating the reaction of the one or more reactants (116,216);
**characterized in that** the film lining (125) is at least one metal and is of a microwave absorbing material for absorbing energy provided by the magnetron (132,232) and transferring the energy to the one or more reactants (116,216) in the form of heat, the film lining (125) contacting the one or more reactants (116,216) received in the lumen (126).

2. The apparatus (110,210) of claim 1, wherein the film lining (125) is of a material that provides a chemical-catalyst for the reaction.

3. The apparatus (110,210) of claim 1, wherein the reaction capillary (112,212) is a capillary tube having a hollow interior defining the lumen (126).

4. The apparatus (110,210) of any one of claims 1 to 3, wherein the film lining (125) is of elemental palladium.

5. The apparatus (110,210) of any one of claims 1 to 3, wherein the film lining (125) comprises a material selected from the group consisting of palladium, silver, copper, nickel, gold, rhodium, and platinum.

6. The apparatus (110,210) of any one of claims 1 to 5, wherein the film lining (125) has a thickness in the range of 2 microns to 10 microns.

7. The apparatus (110,210) of any one of claims 1 to 5, wherein the film lining (125) has a thickness of 6 microns.

8. The apparatus (110,210) of any one of claims 1 to 7, wherein the film lining (125) has a porosity of 75%.

9. The apparatus (110,210) of any one of claims 1 to 8, wherein the film lining (125) comprises grains of 40 nanometers to 60 nanometers in diameter.

10. The apparatus (110,210) of any one of claims 1 to 9, further comprising a reactant supply (130,230) in fluid communication with the lumen (126) of the capillary (112,212).

11. The apparatus (110,210) of claim 10, further comprising a manifold (138,238) coupled downstream of the reactant supply (130,230) and upstream of the capillary (112,212).

12. The apparatus (110,210) of claim 11, wherein the manifold (138,238) has at least one outlet port (140,240) and a plurality of inlet ports (142,242) in fluid communication with said at least one outlet port (140,240).

13. The apparatus (110,210) of claim 12, wherein the reactant supply (130,230) comprises a plurality of reagent reservoirs (144,244) in fluid communication with respective ones of the plurality of inlet ports (142,242) of the manifold (138,238).

14. The apparatus (110,210) of claim 13, further comprising a flow inducer (146,246) for urging the reagent (116,216) from each reservoir (144,244) to the respective inlet ports (142,242).

15. The apparatus (110,210) of any one of claims 1 to 14, therein the reaction capillary (112,212) has a straight cylindrical shape along an axial length extending between upstream and downstream ends of the capillary (112,212).

16. The apparatus (110,210) of any one of claims 1 to 15, wherein the reaction capillary (112,212) has an inner diameter that is less than 1500 microns.

17. A method of reacting a reactant (116,216) comprising:
a) providing a capillary (112,212);
b) passing at least one reactant (116,216) through the capillary (112,212); and
c) irradiating the at least one reactant (116,216) in the capillary (112,212) with microwaves to facilitate a chemical reaction in the capillary (112,212) by which the at least one reactant (116,216) is converted into a product (118,218);
**characterized in that** the capillary (112,212) includes a reaction-enhancing film lining (125) on an inner surface (121) thereof, **in that** the film lining (125) is at least one metal and absorbs energy from the microwaves and transfers the energy to the at least one reactant as heat, and **in that** the film lining (125) contacts the at least one reactant (116,216).

18. The method of claim 17, wherein the film lining (125) provides a chemical catalyst for the reaction.

19. The method of claim 17 or 18, wherein the film lining (125) comprises palladium.

20. The method of any one of claims 17 to 19, wherein the film lining (125) has a thickness of 6 microns.

21. The method of any one of claims 17 to 20, wherein the capillary (112,212) is a capillary tube having a hollow interior defining a lumen (126) through which the reactant (116,216) flows.

## Patentansprüche

1. Reaktorvorrichtung (110, 210), die folgendes umfasst:
a) mindestens eine Reaktionskapillare (112, 212) mit einem Lumen (126) zur Aufnahme von einem oder mehreren Reaktionsteilnehmern (116, 216) für eine Reaktion;
b) ein Magnetron (132, 232) zum Bestrahlen mit Mikrowellen des einen oder der mehreren Reaktionsteilnehmer (116, 216), die sich in zumindest einem Teil der Kapillare (112, 212) befinden; und
c) eine Filmauskleidung (125), die an einer inneren Oberfläche (121) der Kapillare (112, 212) vorgesehen ist, um die Reaktion des einen oder der mehreren Reaktionsteilnehmer (116, 216) zu erleichtern;
**dadurch gekennzeichnet, dass** die Filmauskleidung (125) zumindest ein Metall darstellt und aus einem mikrowellenabsorbierenden Material zur Absorption der durch das Magnetron (132, 232) bereitgestellten Energie besteht, und wobei die Energie in Form von Wärme zu dem einen oder den mehreren Reaktionsteilnehmern (116, 216) übertragen wird, wobei die Filmauskleidung (125) einen Kontakt mit dem einen oder den mehreren in dem Lumen (126) empfangenen Reaktionsteilnehmern (116, 216) herstellt.

2. Vorrichtung (110, 210) nach Anspruch 1, wobei die Filmauskleidung (125) aus einem Material besteht, dass einen chemischen Katalysator für die Reaktion bereitstellt.

3. Vorrichtung (110, 210) nach Anspruch 1, wobei es sich bei der Reaktionskapillare (112, 212) um eine Kapillarröhre mit einem hohlen Inneren handelt, welches das Lumen (126) definiert.

4. Vorrichtung (110, 210) nach einem der Ansprüche 1 bis 3, wobei es sich bei der Filmauskleidung (125) um elementares Palladium handelt.

5. Vorrichtung (110, 210) nach einem der Ansprüche 1 bis 3, wobei die Filmauskleidung (125) ein Material umfasst, das aus der Gruppe ausgewählt wird, die Palladium, Silber, Kupfer, Nickel, Gold, Rhodium und Platin umfasst.

6. Vorrichtung (110, 210) nach einem der Ansprüche 1 bis 5, wobei die Filmauskleidung (125) eine Dicke im Bereich von 2 Mikron bis 10 Mikron aufweist.

7. Vorrichtung (110, 210) nach einem der Ansprüche 1 bis 5, wobei die Filmauskleidung (125) eine Dicke von 6 Mikron aufweist.

8. Vorrichtung (110, 210) nach einem der Ansprüche 1 bis 7, wobei die Filmauskleidung (125) eine Porosität von 75 % aufweist.

9. Vorrichtung (110, 210) nach einem der Ansprüche 1 bis 8, wobei die Filmauskleidung (125) Körner mit einem Durchmesser zwischen 40 Nanometern und 60 Nanometern aufweist.

10. Vorrichtung (110, 210) nach einem der Ansprüche 1 bis 9, wobei diese ferner eine Reaktionsteilnehmerversorgungseinrichtung (130, 230) umfasst, die sich in Fluidkommunikation mit dem Lumen (126) der Kapillare (112, 212) befindet.

11. Vorrichtung (110, 210) nach Anspruch 10, wobei diese ferner einen Verteiler (138, 238) umfasst, der stromabwärts der Reaktionsteilnehmerversorgungseinrichtung (130, 230) und stromaufwärts der Kapillare (112, 212) gekoppelt ist.

12. Vorrichtung (110, 210) nach Anspruch 11, wobei der Verteiler (138, 238) mindestens einen Auslassanschluss (140, 240) und eine Mehrzahl von Einlassanschlüssen (142, 242) in Fluidkommunikation mit dem genannten mindestens einen Auslassanschluss (140, 240) aufweist.

13. Vorrichtung (110, 210) nach Anspruch 12, wobei die Reaktionsteilnehmerversorgungseinrichtung (130, 230) eine Mehrzahl von Reaktionsteilnehmerspeichern (144, 244) in Fluidkommunikation mit entsprechenden Einlassanschlüssen der genannten Mehrzahl von Einlassanschlüssen (142, 242) des Verteilers (138, 238) umfasst.

14. Vorrichtung (110, 210) nach Anspruch 13, wobei diese ferner eine Strömungsinduktionseinrichtung (146, 246) umfasst, um den Reaktionsteilnehmer (116, 216) aus jedem Speicher (144, 244) zu den entsprechenden Einlassanschlüssen (142, 242) zu drängen.

15. Vorrichtung (110, 210) nach einem der Ansprüche 1 bis 14, wobei die Reaktionskapillare (112, 212) eine gerade zylindrische Form entlang einer axialen Länge aufweist, die sich zwischen den stromaufwärts und stromabwärts liegenden Enden der Kapillare (112, 212) erstreckt.

16. Vorrichtung (110, 210) nach einem der Ansprüche 1 bis 15, wobei die Reaktionskapillare (112, 212) einen Innendurchmesser aufweist, der kleiner ist als 1500 Mikron.

17. Verfahren zur Reaktion eines Reaktionsteilnehmers (116, 216), wobei das Verfahren folgendes umfasst:
a) das Bereitstellen einer Kapillare (112, 212);
b) das Leiten mindestens eines Reaktionsteilnehmers (116, 216) durch die Kapillare (112, 212); und
c) das Bestrahlen des mindestens einen Reaktionsteilnehmers (116, 216) in der Kapillare (112, 212) mit Mikrowellen, um eine chemische Reaktion in der Kapillare (112, 212) zu ermöglichen, durch welche der mindestens eine Reaktionsteilnehmer (116, 216) in ein Produkt (118, 218) umgewandelt wird;
**dadurch gekennzeichnet, dass** die Kapillare (112, 212) auf einer inneren Oberfläche (121) eine die Reaktion erleichternde Filmauskleidung (125) aufweist, wobei es sich bei der Filmauskleidung (125) um mindestens ein Metall handelt, und wobei sie die Energie von den Mikrowellen absorbiert und die Energie als Wärme zu dem mindestens einen Reaktionsteilnehmer überträgt, und wobei die Filmauskleidung (125) den mindestens einen Reaktionsteilnehmer (116, 216) berührt.

18. Verfahren nach Anspruch 17, wobei die Filmauskleidung (125) einen chemischen Katalysator für die Reaktion bereitstellt.

19. Verfahren nach Anspruch 17 oder 18, wobei die Filmauskleidung (125) Palladium umfasst.

20. Verfahren nach einem der Ansprüche 17 bis 19, wobei die Filmauskleidung (125) eine dicke von 6 Mikron aufweist.

21. Verfahren nach einem der Ansprüche 17 bis 20, wobei es sich bei der Kapillare (112, 212) um eine Kapillarröhre mit einem hohlen Inneren handelt, welches ein Lumen (126) definiert, durch welches der Reaktionsteilnehmer (116, 216) fließt.

## Revendications

1. Appareil réacteur (110, 210) comprenant :
a) au moins un capillaire de réaction (112, 212) ayant un lumen (126) pour recevoir un ou plusieurs réactifs (116, 216) pour subir une réaction ;
b) un magnétron (132, 232) pour irradier le ou les réactifs (116, 216) contenus dans au moins une partie du capillaire (112, 212) avec des micro-ondes et
c) un revêtement de film (125) prévu sur une surface intérieure (121) du capillaire (112, 212) pour faciliter la réaction du ou des réactifs (116, 216) ;
**caractérisé en ce que** le revêtement de film (125) est au moins un métal et est en un matériau absorbant les micro-ondes pour absorber l'énergie fournie par le magnétron (132, 232) et transférer l'énergie au ou aux réactifs (116, 216) sous la forme de chaleur, le revêtement de film (125) étant en contact avec le ou les réactifs (116, 216) reçus dans le lumen (126).

2. Appareil (110, 210) selon la revendication 1, dans lequel le revêtement de film (125) est d'un matériau qui fournit un catalyseur chimique pour la réaction.

3. Appareil (110, 210) selon la revendication 1, dans lequel le capillaire de réaction (112, 212) est un tube capillaire ayant un intérieur creux définissant le lumen (126).

4. Appareil (110, 210) selon l'une quelconque des revendications 1 à 3, dans lequel le revêtement de film (125) est du palladium élémentaire.

5. Appareil (110, 210) selon l'une quelconque des revendications 1 à 3, dans lequel le revêtement de film (125) comprend un matériau choisi dans le groupe constitué de palladium, de cuivre à l'argent, de nickel, d'or, de rhodium et de platine.

6. Appareil (110, 210) selon l'une quelconque des revendications 1 à 5, dans lequel le revêtement de film (125) a une épaisseur dans la plage comprise entre 2 et 10 microns.

7. Appareil (110, 210) selon l'une quelconque des revendications 1 à 5, dans lequel le revêtement de film (125) a une épaisseur de 6 microns.

8. Appareil (110, 210) selon l'une quelconque des revendications 1 à 7, dans lequel le revêtement de film (125) a une porosité de 75 %.

9. Appareil (110, 210) selon l'une quelconque des revendications 1 à 8, dans lequel le revêtement de film (125) comprend des grains de 40 à 60 nanomètres de diamètre.

10. Appareil (110, 210) selon l'une quelconque des revendications 1 à 9, comprenant en outre une alimentation en réactif (130, 230) en communication fluidique avec le lumen (126) du capillaire (112, 212).

11. Appareil (110, 210) selon la revendication 10, comprenant en outre un collecteur (138, 238) couplé en aval de l'alimentation en réactif (130, 230) et en amont du capillaire (112,212).

12. Appareil (110, 210) selon la revendication 11, dans lequel le collecteur (138, 238) a au moins un orifice de sortie (140, 240) et une pluralité d'orifices d'entrée (142, 242) en communication fluidique avec ledit au moins un orifice de sortie (140, 240).

13. Appareil (110, 210) selon la revendication 12, dans lequel l'alimentation en réactif (130, 230) comprend une pluralité de réservoirs de réactif (144, 244) en communication fluidique avec ceux respectifs de la pluralité d'orifices d'entrée (142, 242) du collecteur (138, 238).

14. Appareil (110, 210) selon la revendication 13, comprenant en outre un inducteur de flux (146, 246) pour pousser le réactif (116, 216) depuis chaque réservoir (144, 244) vers les orifices d'entrée respectifs (142, 242).

15. Appareil (110, 210) selon l'une quelconque des revendications 1 à 14, dans lequel le capillaire de réaction (112, 212) a une forme cylindrique droite le long d'une longueur axiale s'étendant entre les extrémités amont et aval du tube capillaire (112, 212).

16. Appareil (110, 210) selon l'une quelconque des revendications 1 à 15, dans lequel le capillaire de réaction (112, 212) a un diamètre intérieur qui est inférieur à 1 500 microns.

17. Procédé de réaction d'un réactif (116, 216) comprenant les étapes consistant à :
a) fournir un capillaire (112, 212) ;
b) faire passer au moins un réactif (116, 216) à travers le capillaire (112, 212) ; et
c) irradier l'au moins un réactif (116, 216) dans le capillaire (112, 212) avec des micro-ondes pour faciliter une réaction chimique dans le capillaire (112, 212) par quoi l'au moins un réactif (116, 216) est converti en un produit (118, 218) ;
**caractérisé en ce que** le capillaire (112, 212) comprend un revêtement de film renforçant la réaction (125) sur une surface intérieure (121) de celui-ci, **en ce que** le revêtement de film (125) est au moins un métal et absorbe l'énergie des micro-ondes et transfère l'énergie à l'au moins un réactif sous forme de chaleur, et **en ce que** le revêtement de film (125) est en contact avec l'au moins un réactif (116, 216).

18. Procédé selon la revendication 17, dans lequel le revêtement de film (125) fournit un catalyseur chimique pour la réaction.

19. Procédé selon la revendication 17 ou 18, dans lequel le revêtement de film (125) comprend du palladium.

20. Procédé selon l'une quelconque des revendications 17 à 19, dans lequel le revêtement de film (125) a une épaisseur de 6 microns.

21. Procédé selon l'une quelconque des revendications 17 à 20, dans lequel le capillaire (112, 212) est un tube capillaire ayant un intérieur creux définissant un lumen (126) à travers lequel le réactif (116, 216) s'écoule.
